# EUROPÄISCHE PATENTSCHRIFT

(11) **EP 3 128 925 B1**
(45) Veröffentlichungstag und Bekanntmachung des Hinweises auf die Patenterteilung: **14.08.2019**
(21) Anmeldenummer: 15713535.1
(22) Anmeldetag: 07.04.2015
(51) Int. Cl.: A61B 17/02

(54) **VORRICHTUNG ZUR VERMINDERUNG DER RETRAKTION EINER FASZIE ODER EINES WEICHTEILMANTELS BEI EINEM OFFENEN WEICHTEILDEFEKT**
DEVICE FOR REDUCING THE RETRACTION OF A FASCIA OR A SOFT TISSUE MANTLE IN AN OPEN SOFT TISSUE DEFECT
DISPOSITIF DE RÉDUCTION DE LA RÉTRACTION D'UNE APONÉVROSE OU D'UNE ENVELOPPE DE PARTIES MOLLES EN CAS DE DÉFAUT D'OUVERTURE DE PARTIES MOLLES

(30) Priorität: 10.04.2014 DE 102014206959; 26.05.2014 DE 102014209995
(43) Veröffentlichungstag der Anmeldung: 15.02.2017
(60) Teilanmeldung: 19181115.7
(73) Patentinhaber: Fasciotens GmbH, 45138 Essen (DE)
(72) Erfinder: Lill, Gereon, 50931 Köln (DE)
(74) Vertreter: Wolff, Felix
(86) Internationale Anmeldenummer: PCT/EP2015/057495
(87) Internationale Veröffentlichungsnummer: WO 2015/155176

(56) Entgegenhaltungen:
- WO-A1-94/06354
- WO-A1-2013/092938
- JP-A- H0 871 073
- US-A- 3 572 326
- US-A- 5 782 746
- US-A- 5 876 333
- US-A1- 2007 156 028
- US-A1- 2010 191 253
- US-B1- 6 387 047
- US-E- R E32 021

## Beschreibung

### Gebiet der Erfindung

Die Erfindung betrifft eine Vorrichtung und ein Verfahren zur Verminderung der Retraktion der Ränder einer geöffneten Faszie eines Patienten mit einem offenen Weichteildefekt, insbesondere mit einer offenen Bauchdecke oder einem offenen Weichteildefekt am Rücken. Ferner betrifft die Erfindung ein Kit zur Behandlung eines offenen Weichteildefekts, insbesondere einer geöffneten Bauchdecke oder einem offenen Weichteildefekt am Rücken, mit mindestens einem Schwamm zum Ausfüllen eines Weichteildefekts, insbesondere einer Öffnung in der Bauchdecke oder eines offenen Weichteildefekts am Rücken.

Beschrieben wird ferner ein Schwamm für die Unterdruck-Wundtherapie.

Die Erfindung kann bei offenen Weichteildefekten Anwendung finden. Solche Weichteildefekte können beispielsweise als Bauchwanddefekt, Rückenwanddefekt, Weichteildefekt im Bereich der Hüfte oder Weichteildefekt an den Gliedmaßen vorliegen.

### Stand der Technik

Im Stand der Technik ist es bekannt, bei chirurgischen Operationen Retraktionsvorrichtungen zu verwenden, über welche ein Weichteildefekt während einer Operation offengehalten werden kann. Derartige Retraktionsvorrichtungen bewirken eine Retraktion der Ränder einer geöffneten Faszie eines Patienten und sind beispielsweise aus US R E32 021, US 5 782 746 A, US 5 876 333 A, US 3 572 326 A, US 6 387 047 B1, WO 2013/092 938 A1 und WO 94/06 354 A1 bekannt. Ferner beschreibt die US 2010/191 253 A1 eine Vorrichtung zum Spannen einer Hautoberfläche während einer Haartransplantation und US 2007/156 028 A1 eine Vorrichtung zum Offenhalten des Mundbereichs während einer Operation im Mundraum. Zudem beschreibt die JP H08 71073 A eine Vorrichtung zum Anheben einer geschlossenen Bauchwand während eines laparoskopischen chirurgischen Eingriffs.

Zur Behandlung einiger Baucherkrankungen, welche mit einer intraabdominellen Druckerhöhung, einem abdominellen Kompartmentsyndrom, einhergehen, kann eine Eröffnung der Bauchdecke erforderlich werden, um den Bauchraum freizulegen und einem Anstieg des intraabdominalen Drucks entgegenzuwirken. Dieser Zustand wird auch als "offenes Abdomen" bezeichnet. Die Bauchdecke bleibt dabei üblicherweise über einen Zeitraum von mehreren Tagen bis hin zu einigen Monaten weit geöffnet. Auch bei andersartigen Diagnosen ohne intraabdominelle Druckerhöhung kann ein offenes Abdomen notwendig sein bzw. vorliegen.

In der Regel wird die Bauchdecke über einen provisorischen abdominellen Verschluss nach Art eines Vakuumverbands temporär abgedichtet, so dass eine Unterdruck-Wundtherapie durchgeführt werden kann. Hierbei wird ein schwammartiges Material in die Lücke in der Bauchdecke eingesetzt und das offene Abdomen über Folien abgedichtet. Über den Schwamm wird ein Unterdruck angelegt und überschüssige Flüssigkeit aus dem Bauchraum abgesaugt. Alternativ kann die Wunde auch ohne eine Unterdruck-Wundtherapie versorgt werden.

Die Therapie mittels offenem Abdomen hat sich als besonders vorteilhaft für die Heilung von Erkrankungen des Bauchraums erwiesen. Die Eröffnung der Bauchdecke bringt aber den Nachteil mit sich, dass sich die Ränder der in der Bauchdecke liegenden geöffneten Faszie während der über Tage bzw. Wochen anhaltenden Öffnung des Abdomens in Richtung der Seiten zurückziehen können. Dadurch wird das Schließen der Bauchdecke und damit der Faszie nach Normalisierung des intraabdominellen Drucks erschwert bzw. unmöglich. Eine primäre Wundheilung, bei welcher die Wundränder aneinander anliegen, ist oftmals nicht möglich. Auch ein sekundärer, schichtgerechter Wundverschluss kann oftmals nicht erreicht werden. Es kann zu einer lang anhaltenden sekundären Wundheilung kommen, die mit der Bildung von Narbengewebe einhergeht. Oftmals heilt die Wunde in einem Narbenbruch ab.

Um die Zeit bis zu einem dauerhaften Verschluss der Bauchdecke zu überbrücken und die Ränder der Faszie aneinander anzunähern, wird die Bauchdecke nach erfolgter Therapie mittels offenem Abdomen oftmals nur provisorisch verschlossen. Aus der DE 36 31 762 A1 ist z.B. ein provisorischer Verschluss für eine Bauchdeckenöffnung bekannt, über welche die Bauchdecke vorübergehend geschlossen werden kann und dabei eine Spannung auf die Ränder der Faszie ausgeübt wird. Die DE 4034 705 A1 offenbart einen weiteren provisorischen Verschluss, bei welchem eine Spannung auf die Ränder der geöffneten Faszie aufgebracht wird. Ferner beschreibt die DE 34 44 782 C2 einen Gleitverschluss, der an die Ränder der geöffneten Faszie angenäht werden kann, um diese zu verschließen.

Bei den zuvor genannten provisorischen Bauchdeckenverschlüssen werden die Ränder der geöffneten Faszie gespannt, so dass ein nachträglicher dauerhafter Verschluss der Faszie mit primärer Wundheilung ermöglicht wird. Die Faszienränder werden bei diesen Verschlüssen aufeinander zu gezogen, so dass diese aneinander anliegen. Da der Bauchraum bei diesen provisorischen Verschlüssen nicht geöffnet ist, können sich die Innereien des Patienten nicht ausdehnen, was jedoch für Patienten mit einem Anstieg des intraabdominellen Drucks lebensnotwendig ist. Die Bauchdecke muss oftmals wegen der retrahierten Faszien nach der ohnehin schon längeren Periode des offenen Abdomens über eine längere Zeitspanne nur provisorisch verschlossen bleiben bevor der dauerhafte Verschluss der Bauchdecke erfolgen kann. Oftmals gelingt dieser nur mit Hilfe alloplastischer Materialien.

Aus der Veröffentlichung "Sekundärverschluß von Hautdefekten unter Anwendung von Gummizügen (dynamische Sekundärnaht), Fankhauser et al., Chirurg (1995) 66, 1154-1157, ISSN 0009-4722 ist ein sekundärer Wundverschluss für einen Hautdefekt im Bereich der Gliedmaßen bekannt, bei dem die Hautränder über transcutan oder intracutan fixierte Gummizüge aufeinander zu gezogen werden. Mit diesem Verfahren ist es zwar möglich, die Haut zu dehnen, um einen sekundären Verschluss der Haut zu ermöglichen, nachteilig ist jedoch, dass eine Verminderung der Retraktion der unter der Haut liegenden Faszien, insbesondere während des Bestands der Faszienöffnung, nicht erreicht werden kann. Ein weiterer Nachteil dieser Vorrichtung besteht darin, dass sich die Vorrichtung auf den Hauträndern abstützt, an welchen gezogen wird, wodurch der unter dem Hautdefekt liegende Raum verkleinert wird.

Die zuvor genannten Nachteile ergeben sich auch bei anderen offenen Weichteildefekten, wie beispielsweise einer geöffneten Rückenwand oder einem offenen Weichteildefekt im Bereich der Hüfte oder an den Gliedmaßen. Im Bereich des Rückens kann es beispielsweise nach einer Operation erforderlich werden, die Rückenwand zur Infektbehandlung geöffnet zu belassen, wobei es ebenfalls zu einer Retraktion der Faszien kommen kann.

### Offenbarung der Erfindung

Vor diesem Hintergrund liegt der Erfindung die Aufgabe zugrunde, den dauerhaften Verschluss des Weichteildefekts, insbesondere der Bauchdecke oder der Rückenwand, unmittelbar nach einer Therapie mit einem offenen Weichteildefekt, insbesondere mit einem offenen Abdomen oder einer offenen Rückenwand, zu ermöglichen.

Die Aufgabe wird gelöst durch eine Vorrichtung mit den Merkmalen des Patentanspruchs 1.

Durch die auf die Faszie wirkende Zugkraft werden die Ränder der geöffneten Faszie auf Abstand gehalten, so dass der Zugang zu dem unter dem Weichteildefekt liegenden Körperinnenraum, insbesondere zum Bauchraum, möglich ist, beispielsweise eine Unterdruck-Wundtheraphie durchgeführt werden kann oder sich die Innereien durch den Weichteildefekt, insbesondere die Bauchdeckenöffnung oder einen Weichteildefekt am Rücken, hindurch ausdehnen können. Zudem wird durch die Zugkraft die Spannung der Faszie aufrechterhalten und damit die Retraktion der Ränder der Faszie zumindest vermindert. Die Ränder der Faszie können nach Abschluss der Therapie mittels offenem Weichteildefekt, insbesondere mittels offenem Abdomen oder offenem Weichteildefekt am Rücken, aneinander angelegt und direkt verschlossen werden, wodurch eine verbesserte Wundheilung beim dauerhaften Verschluss des Weichteilgewebes, insbesondere der Bauchdecke oder der Rückenwand, ermöglicht wird.

Durch die erfindungsgemäße Vorrichtung kann die Faszie derart weit offen gehalten werden, dass der unter dem Weichteildefekt liegende Körperinnenraum, insbesondere der Bauchraum, frei zugänglich ist und/oder die innenliegenden Organe durch den Hautdefekt, insbesondere die Bauchdeckenöffnung oder die Rückenwandöffnung, zumindest im Wesentlichen ungehindert austreten können. Dies bringt den Vorteil mit sich, dass eine ungehinderte Entspannung eines Überdrucks im Körper, insbesondere im Bauchraum oder im Raum unterhalb der Rückenwand, bzw. der ungehinderte Austritt überschüssiger Flüssigkeit aus dem Körper, insbesondere dem Bauchraum oder dem Raum unterhalb der Rückenwand, möglich ist. Erfindungsgemäß werden die Ränder der geöffneten Faszie durch die Zugkraft derart gehalten, dass sie nicht aneinander anliegen. Die Vorrichtung ist bevorzugt derart ausgebildet, dass kein in der Ebene der Körperoberfläche, insbesondere der Bauchdecke oder Rückenwand, angeordnetes Verbindungsmittel zwischen den Faszienrändern angeordnet ist.

Die erfindungsgemäße Vorrichtung ermöglicht eine Volumenzunahme des ursprünglich unter dem Weichteildefekt liegenden Körperinnenraums durch Ausdehnung in den ursprünglich von dem Weichteilgewebe eingenommen Raum.

Besonders bevorzugt ist die über die Vorrichtung aufgebrachte Zugkraft derart ausgerichtet, dass die Ränder der geöffneten Faszie nicht aufeinander zu gezogen werden. Insofern wird die Zugkraft bevorzugt derart auf die Ränder der Faszie aufgebracht, dass der offene Weichteildefekt nicht verkleinert wird. Vorteilhafterweise ist die Zugkraft derart ausgerichtet, dass der durch die Ränder der Faszie begrenzte Raum vergrößert wird.

Die Vorrichtung kann derart ausgebildet sein, dass die Zugkraft an der Faszie des Abdomens im Wesentlichen in ventraler Richtung oder in ventrolateraler Richtung oder in lateraler Richtung angreift. Alternativ kann die Vorrichtung derart ausgebildet sein, dass die Zugkraft an der Faszie des Rückens in dorsaler oder dorsolateralter Richtung angreift. Bevorzugt ist zumindest eine Komponente der Zugkraft quer, insbesondere senkrecht, zu einer virtuellen Verbindungslinie zwischen den Rändern der Körperöffnung, insbesondere geöffneten Bauchdecke oder geöffneten Rückenwand, bzw. der geöffneten Faszie, ausgerichtet. Die auf die Ränder der Faszie wirkende Zugkraft weist somit eine quer, insbesondere senkrecht, zur Körperöffnung, insbesondere Bauchdeckenöffnung oder Rückenwandöffnung, vom Patienten weg verlaufende Komponente auf. Der Rand der Faszie kann aber auch umgestülpt und dann auf Spannung gehalten werden. In diesem Fall ist die Zugkraft im Wesentlichen parallel zur Körperoberfläche, insbesondere zur Bauchdecke oder Rückenwand, des Patienten aber von der Körperöffnung, insbesondere der Bauchdeckenöffnung oder Rückenwandöffnung, weg gerichtet.

Gemäß einer vorteilhaften Ausgestaltung ist die Vorrichtung derart ausgebildet, dass die Zugkraft an der Faszie in einer Richtung angreift, die mit einer virtuellen Verbindungslinie zwischen den Rändern der geöffneten Faszie einen Winkel einschließt, der im Bereich von 5° bis 90° oder im Bereich von 10° bis 80° oder im Bereich von 15° bis 70 ° liegt. Bevorzugt liegt der Winkel im Bereich von 30° bis 60°, besonders bevorzugt im Bereich von 35° bis 55°. Beispielsweise kann der Winkel im Wesentlichen 45° betragen.

Gemäß der Erfindung weist die Vorrichtung zumindest ein mit der Faszie verbindbares Zugorgan auf. Über das Zugorgan kann die Zugkraft in die Faszie eingeleitet werden. Bevorzugt sind mehrere Zugorgane vorgesehen, über welche Zugkräfte an mehrere verschiedenen Stellen auf die Ränder der geöffneten Faszie aufgebracht werden können. Die verschiedenen Zugorgane können jeweils unterschiedlich starke und unterschiedlich ausgerichtete Zugkräfte in die Faszie einleiten. Alternativ kann das Zugorgan mit der Körperoberfläche, insbesondere mit der Bauchdecke oder Rückenwand, einschließlich der Faszie, verbunden sein, so dass nicht nur die Faszie, sondern auch die über der Faszie liegende Haut auf Spannung gehalten wird.

Bevorzugt ist das Zugorgan als Faden und/oder als Draht und/oder als Netz ausgebildet. Es können herkömmliche, in der Chirurgie verwendete Fäden, Drähte oder Netze zur Anwendung kommen. Besonders vorteilhaft ist es, wenn das Zugorgan ein Federmittel aufweist, welches in Richtung der Zugkraft vorgespannt werden kann, so dass das Zugorgan auf Spannung gehalten werden kann. Alternativ oder zusätzlich kann das Zugorgan elastisch ausgebildet sein und in Richtung der Zugkraft vorgespannt werden. Das Zugorgan kann direkt oder indirekt mittels eines Verbindungsmittels an der Faszie angebunden sein. Bevorzugt ist das Zugorgan als eine Kombination eines Fadens und/oder eines Drahts und/oder eines Netzes ausgebildet.

Bevorzugt ist es ferner, wenn das Zugorgan über ein flächig ausgebildetes Verbindungsmittel mit der Faszie verbindbar ist. Über das flächige Verbindungsmittel kann eine verbesserte Anbindung des Zugorgans an die Faszie erreicht werden. Die Gefahr des Ausreißens der Faszie kann vermindert werden und die Faszie kann beim wiederholten Wechseln des Zugorgans geschont werden. Besonders bevorzugt ist eine Ausgestaltung, bei welcher das flächige Verbindungsmittel mit der Faszie vernähbar ist. Alternativ kann das Verbindungsmittel beispielsweise Haken, insbesondere Widerhaken, aufweisen, welche in die Faszie eingebracht werden können. Das Verbindungsmittel kann beispielsweise als Netz oder als Platte ausgebildet sein. Das Zugorgan kann über das flächig ausgebildete Verbindungsmittel mit der Faszie und zusätzlich mit anderen Bestandteilen des Weichteilmantels, bevorzugt mit dem gesamten Weichteilmantel, verbindbar sein. Beispielsweise kann neben der Faszie auch die Haut mit dem flächigen Verbindungsmittel verbunden sein.

Vorteilhaft ist es, wenn das flächig ausgebildete Verbindungsmittel reversibel mit dem Zugorgan verbindbar ist, so dass das Zugorgan und/oder die Aufhängung problemlos entfernt und wieder angebracht werden kann, um den offenen Weichteildefekt, insbesondere das offene Abdomen oder den offenen Rücken, zu behandeln.

Erfindungsgemäß weist die Vorrichtung eine außerhalb des Körpers des Patienten anordbare Aufhängung auf, an welcher das Zugorgan anbindbar ist. Die Aufhängung kann dem Weichteildefekt, insbesondere der Bauchdeckenöffnung oder Rückenwandöffnung, des Patienten vorgelagert sein. Es ist möglich, ein Ende des Zugorgans an der Aufhängung und das gegenüberliegende Ende des Zugorgans an der Faszie anzubinden. Alternativ können beide Enden des Zugorgans an der Faszie angebunden sein und das Zugorgan über ein an der Aufhängung angeordnetes Umlenkmittel geführt sein. Beide Enden des Zugorgans können an demselben Randabschnitt der geöffneten Faszie oder an unterschiedlichen Randabschnitten der geöffneten Faszie angebunden werden.

Gemäß einer vorteilhaften Ausgestaltung sind die Zugorgane derart an die Aufhängung angebunden, dass über die Zugorgane eine Zugkraft auf die Ränder der Faszie aufgebracht wird, die in einer Richtung angreift, die mit einer virtuellen Verbindungslinie zwischen den Rändern der geöffneten Faszie einen Winkel einschließt, der im Bereich von 5° bis 90° oder im Bereich von 10° bis 80° oder im Bereich von 15° bis 70 ° liegt. Bevorzugt liegt der Winkel im Bereich von 30° bis 60°, besonders bevorzugt im Bereich von 35° bis 55°. Beispielsweise kann der Winkel im Wesentlichen 45° betragen.

Als vorteilhaft hat es sich ferner erwiesen, wenn die Zugkraft über eine insbesondere an der Aufhängung angeordnete Spannvorrichtung einstellbar ist. Die Spannvorrichtung kann manuell betätigbar sein, so dass die Spannung einzelner Zugorgane oder die Spannung einer Gruppe von Zugorganen während des Bestehens des Weichteildefekts, insbesondere des geöffneten Abdomens oder der geöffneten Rückenwand, eingestellt werden kann. Somit wird es möglich, auf Veränderungen der Faszie zu reagieren und ggf. die Spannung zu justieren. Alternativ kann die Spannvorrichtung derart ausgebildet sein, dass sie das Zugorgan automatisch nachspannt, sobald eine Entspannung des Zugorgans eintritt. Die Spannvorrichtung kann einen Kraftverstärker, beispielsweise einen Hebelkraftverstärker, aufweisen, so dass die Zugkraft durch eine im Vergleich zu der Zugkraft kleinere Einstellkraft eingestellt werden kann.

Bevorzugt ist die Spannvorrichtung derart ausgestaltet, dass die auf die Faszie einwirkende Zugkraft eingestellt werden kann, ohne das Zugorgan von der Faszie und/oder der Aufhängung zu lösen. Vorteilhaft ist es, wenn jedem Zugorgan eine eigene Spannvorrichtung zugeordnet ist, so dass die Spannung der Zugorgane individuell eingestellt werden kann. Alternativ oder zusätzlich kann einer Gruppe von Zugorganen eine Spannvorrichtung zugeordnet sein, so dass die Spannung der Zugorgane der Gruppe gemeinsam eingestellt werden kann.

Vorteilhaft ist es ferner, wenn die Vorrichtung eine Schutzeinrichtung aufweist, die ein Zugorgan oder mehrere Zugorgane bei Überschreiten einer vorgegebenen Zugkraft in einen Schutzzustand verbringt, in dem die über das Zugorgan auf die Faszie aufgebrachte Zugkraft reduziert ist. Dies bringt den Vorteil mit sich, dass die Faszie vor dem Aufbringen zu großer Zugkräfte geschützt werden kann. In dem Schutzzustand kann das Zugorgan von der Faszie und/oder der Aufhängung gelöst sein. Ferner ist es möglich, dass das Zugorgan in dem Schutzzustand entspannt ist.

Eine bevorzugte Ausgestaltung sieht vor, dass die Aufhängung vertikal, also parallel zu einer gedachten Linie zwischen Scheitel und Sohle des Patienten, entlang des Abdomens oder entlang des Rückens verlaufend anordbar ist. Die Aufhängung kann vom Bereich des Brustbeins bis in den Bereich des vorderen Beckenrings angeordnet werden, so dass eine kranio-kaudal verlaufende Aufhängung gebildet wird. Die Aufhängung kann eine oder mehrere, insbesondere vertikal verlaufende, Stangen aufweisen. Alternativ ist es möglich, die Aufhängung nach Art eines Rahmens auszubilden, welcher um den Weichteildefekt, insbesondere die Öffnung der Bauchdecke herum angeordnet werden kann. Bei einer Aufhängung mit Stangen verlaufen die Stangen bevorzugt gerade oder gekrümmt. Vorteilhafterweise haben die Stangen einen eckigen oder runden Querschnitt.

Die Aufhängung ist an den Körper des Patienten anbindbar, so dass sich die Aufhängung bei einer Lageänderung des Patienten mit diesem mitbewegen kann. Die Anbindung kann über eine am Körper des Patienten festlegbare Befestigungseinrichtung, insbesondere eine Gurtbefestigung oder eine Befestigungskette, erfolgen. Es können mehrere Befestigungseinrichtung vorgesehen sein. Bevorzugt ist eine Ausgestaltung mit zwei Befestigungseinrichtungen, die im oberen (kranialen) und unteren (kaudalen) Bereich des Abdomens oder des Rückens angeordnet werden können.

Bevorzugt ist die Befestigungseinrichtung derart ausgebildet, dass sie auf die Haut des Patienten aufgelegt werden kann oder an einem Knochen des Patienten befestigt werden kann. Die Befestigung kann beispielsweise am Brustbein und/oder am vorderen Beckenring erfolgen. Zur Befestigung der Befestigungseinrichtung an einem Knochen kann die Befestigungsvorrichtung bevorzugt in dem Knochen fixiert werden, beispielsweise dadurch, dass die Befestigungseinrichtung und/oder ein mit der Befestigungseinrichtung verbundenes Verbindungmittel in den Knochen eingeschraubt oder eingesteckt oder eingetrieben wird.

Eine vorteilhafte Ausgestaltung sieht vor, dass die Befestigungseinrichtung als Korsett ausgebildet ist, welches an dem Körper des Patienten festlegbar ist.

Bevorzugt ist die Befestigungseinrichtung der Vorrichtung derart vorgesehen, dass sie auf die Haut in einem Bereich in der Umgebung des Weichteildefekts auflegbar ist, in welchem durch die Vorrichtung keine Zugkräfte auf die unter der Haut liegende Faszie aufgebracht werden

Die Erfindung sieht vor, dass die Befestigungseinrichtung ein auf die Haut des Patienten auflegbares Druckverteilungsmittel aufweist. Das Druckverteilungsmittel ist bevorzugt mit einem Medium füllbar. Als Medium zum Füllen des Druckverteilungsmittels kann ein Gas, insbesondere Luft, eine Flüssigkeit, insbesondere Wasser, oder ein Gel verwendet werden. Das Druckverteilungsmittel kann eine Kammer aufweisen, in welche das Medium einbringbar ist. Über das Druckverteilungsmittel können auf die Haut des Patienten wirkenden Druckkräfte gleichmäßig verteilt werden, so dass die Gefahr der Bildung von Druckstellen auf der Haut verringert wird. Besonders bevorzugt weist die Befestigungseinrichtung ein Druckverteilungsmittel mit mehreren, insbesondere zwei, Kammern auf. Vorteilhaft ist es, wenn die Kammern derart ausgebildet sind, dass sie wechselweise mit einem Medium gefüllt werden können, so dass die Druckkräfte abwechselnd in verschiedene Bereiche der Haut eingeleitet werden können. Die Kammern sind bevorzugt nach Art von konzentrischen Kreisen ausgebildet, wobei eine erste Kammer als ein Kreisring ausgebildet ist, der eine zweite Kammer umschließt.

Gemäß der Erfindung weist die Befestigungseinrichtung ein Implantat, auf, welches an einem Knochen des Patienten befestigbar ist. Das Implantat kann als Fixateur interne ausgebildet sein. Das Implantat ist besonders bevorzugt ein bereits im Körper des Patienten vorhandenes Implantat, wie beispielsweise ein Wirbelsäulenimplantat. Es ist alternativ möglich, das Implantat zusammen mit der Vorrichtung zur Verminderung der Retraktion der Faszien einzubringen. Das Implantat weist mehrere Längsträger auf. Die Längsträger des Implantats sind an Wirbelkörpern der Wirbelsäule des Patienten befestigbar. Zur Befestigung des Implantats, insbesondere der Längsträger, an den Knochen umfasst das Implantat bevorzugt ein oder mehrere Verbindungsmittel, insbesondere eine oder mehrere Schrauben.

Die Erfindung sieht vor, dass die Vorrichtung eine Kraftmesseinrichtung zur Messung der durch das Zugorgan auf die Faszie aufgebrachten Zugkraft aufweist. Die Kraftmesseinrichtung kann derart ausgestaltet sein, dass die Summe der von verschiedenen Zugorganen aufgebrachten Zugkräfte messbar ist. Besonders bevorzugt weist die Vorrichtung mehrere Kraftmesseinrichtungen zur Messung der durch verschiedene Zugorgane aufgebrachten Zugkräfte auf. Die Kraftmesseinrichtung kann derart ausgestaltet sein, dass jeweils die durch ein Zugorgan aufgebrachte Zugkraft oder die durch eine Gruppe von Zugorganen aufgebrachte Zugkraft messbar ist. Die Kraftmesseinrichtung kann einen Federkraftmesser, einen induktiver Kraftmesser, einen kapazitiver Kraftmesser, einen optischer Kraftmesser, einen Dehnungsmessstreifen, einen magnetischen Kraftmesser, einen elektromagnetischen Kraftmesser oder einen piezoelektrischen Kraftmesser aufweisen. Die Kraftmesseinrichtung kann mit einem Zugorgan verbunden sein, insbesondere derart, dass die Kraftmesseinrichtung im Kraftfluss der Zugkraft angeordnet ist. Alternativ oder zusätzlich kann die Kraftmesseinrichtung zwischen der Aufhängung und der Befestigungseinrichtung, insbesondere in einer zwischen der Aufhängung und der Befestigungseinrichtung vorgesehenen Stütze, angeordnet sein. Ferner ist es möglich, dass die Kraftmesseinrichtung als Teil der Befestigungseinrichtung ausgebildet ist, welche ein mit einem Medium, beispielsweise einem Gel, füllbares Druckverteilungsmittel aufweist. Durch Messung der Kompression des Mediums kann die auf dem Druckverteilungsmittel lastende Kraft bestimmt werden.

Besonders bevorzugt ist die Befestigungseinrichtung derart ausgebildet, dass sie an das Gewebe, insbesondere die Haut des Patienten angenäht werden kann.

Vorteilhaft ist es, wenn die Aufhängung über mehrere Befestigungseinrichtungen an den Körper des Patienten anbindbar ist, wobei die Befestigungseinrichtungen unterschiedlich ausgebildet sind. Beispielsweise kann im oberen Bereich des Abdomens oder des Thorax eine Befestigungseinrichtung nach Art einer Gurtbefestigung und im unteren Bereich des Abdomens eine Befestigungseinrichtung, die an einem Knochen des Patienten befestigt werden kann, verwendet werden. Alternativ können die Befestigungseinrichtungen identisch ausgebildet sein. Beispielsweise können zwei als Gurtbefestigung ausgebildete Befestigungseinrichtungen, insbesondere eine erste Befestigungseinrichtung im oberen Bereich des Abdomens oder des Thorax und eine zweite Befestigungseinrichtung im unteren Bereich des Abdomens, vorgesehen sein. Eine weitere Alternative besteht darin, dass nur eine einzelne Befestigungseinrichtung vorgesehen ist. Die einzelne Befestigungseinrichtung kann entweder im oberen Bereich des Abdomens oder des Rückens oder im unteren Bereich des Abdomen oder Rückens anordbar sein.

Eine besonders vorteilhafte Ausgestaltung sieht vor, dass die Aufhängung gegenüber der Befestigungseinrichtung beweglich, insbesondere schwenkbeweglich, gelagert ist. Durch eine derartige Lagerung können Bewegungen des Patienten, die z.B. durch die Atmung und/oder beim Husten entstehen können, ausgeglichen werden. Die Aufhängung kann bevorzugt insgesamt gegenüber dem Patienten bewegbar angeordnet sein. Alternativ kann die Aufhängung starr mit der Befestigungseinrichtung gekoppelt sein und/oder einstückig mit der Befestigungseinrichtung ausgebildet sein.

Die Aufhängung kann beispielsweise mit der Befestigungseinrichtung über mindestens ein Gelenk gekoppelt sein, welches vorteilhafterweise als Drehgelenk oder als Kugelgelenk ausgebildet ist. Es ist möglich, mehrere Gelenke an der Aufhängung vorzusehen, so dass sich die Aufhängung gegenüber mehreren Befestigungseinrichtungen bewegen kann. Bei einer nach Art eines Rahmens ausgebildeten Aufhängung mit mehreren Rahmenabschnitten können die Rahmenabschnitte über Gelenke miteinander verbunden werden, um Bewegungen der Rahmenanschnitte gegeneinander zuzulassen. Alternativ kann die Aufhängung über Federelemente, beispielsweise über federnde Teleskopstangen, mit der Befestigungseinrichtung gekoppelt sein.

Bevorzugt ist die Aufhängung kraftschlüssig mit der Befestigungseinrichtung verbunden.

Bevorzugt ist es ferner, wenn die Aufhängung an den Körper des Patienten anpassbar ist, so dass die Vorrichtung bei Patienten mit unterschiedlichem Körperbau einsetzbar ist. Die Aufhängung kann teleskopierbar und/oder aus mehreren Modulen zusammensetzbar ausgebildet sein, so dass die Abmessungen der Aufhängung je nach Körperbau des Patienten eingestellt werden können.

In diesem Zusammenhang ist es besonders bevorzugt, wenn die Länge der Aufhängung, insbesondere in einer vertikal entlang des Abdomens oder des Rückens verlaufenden Richtung, einstellbar ist. Die Länge der Aufhängung kann beispielsweise über ein teleskopierbares Element, insbesondere eine Teleskopstange, eingestellt werden. Die Teleskopstange kann eine Einrastvorrichtung aufweisen, über welche die Länge der Teleskopstange festgestellt werden kann.

Ferner ist es von Vorteil, wenn die Position der Aufhängung gegenüber dem Körper eingestellt werden kann. Zur Veränderung der Stellung der Aufhängung kann ein teleskopierbares Element zum Einsatz kommen.

Alternativ zu einer Anbindung der Aufhängung am Patienten kann die Aufhängung an eine patientenunabhängige Halterung, insbesondere ein Bett oder eine Liege oder eine sich am Bodenabstützende Halterung, anbindbar sein. Bei der Anbindung an eine patientenunabhängige Halterung muss gewährleistet sein, dass die Aufhängung bei einer Lageänderung des Patienten mit diesem mitbewegt werden kann, um die Zugkraft auf die Faszie unverändert aufrecht erhalten zu können.

Bevorzugt ist die Aufhängung wahlweise entweder an den Patienten oder an eine patientenunabhängige Halterung, insbesondere ein Bett oder eine Liege, anbindbar, so dass bedarfsweise zwischen diesen beiden Arten der Aufhängung hin-und her gewechselt werden kann oder beide Arten der Aufhängung gleichzeitig genutzt werden können. Besonders bevorzugt ist die patientenunabhängige Halterung als Galgenverlängerung, an ein Bett angebundene Bettanhängung oder als sich am Boden abstützende Halterung ausgebildet.

Beschrieben wird ferner ein Schwamm für die Unterdruck-Wundtherapie, welcher einen im Wesentlichen dreieckigen oder im Wesentlichen trapezförmigen oder im Wesentlichen halbrunden oder im Wesentlichen halbovalen oder im Wesentlichen halbelliptischen Querschnitt aufweist. Alternativ kann der Schwamm einen rechteckigen Querschnitt aufweisen mit einem Verhältnis der Höhe zur Breite im Bereich von 1/1 bis 1/5. Bevorzugt ist der Schwamm derart ausgebildet, dass er einen Bereich zwischen den Rändern einer geöffneten Faszie und einer auf die Faszie einwirkenden Vorrichtung zur Verminderung der Retraktion der Ränder einer geöffneten Faszie ausfüllt.

Gemäß einer bevorzugten Ausgestaltung weist der Schwamm einen innenliegenden Bereich mit gegenüber seiner Oberfläche erhöhter Festigkeit oder eine innenliegende, nicht deformierbare Grundform auf. Dies bringt den Vorteil mit sich, dass der Schwamm eine erhöhte Stabilität aufweist.

Beschrieben wird ferner ein Schwamm für die Unterdruck-Wundtherapie, welcher eine Ausnehmung zur Aufnahme eines insbesondere langgestreckten Gegenstands, beispielsweise einer Aufhängung, aufweist und/oder dessen Kontur an die Außenkontur der Aufhängung angepasst ist. Über den Schwamm kann die Aufhängung der vorstehend beschriebenen Vorrichtung abgedeckt werden, so dass eine anschließende Abdichtung über ein Dichtmittel, beispielsweise eine Folie, erfolgen kann. Der Schwamm kann einen Querschnitt aufweisen, der im Wesentlichen die Form eines Sektors eines Kreisrings hat.

Bei einem Kit der eingangs genannten Art wird zur Lösung der Aufgabe vorgeschlagen, eine vorstehend beschriebene Vorrichtung zur Verminderung der Retraktion der Ränder einer geöffneten Faszie eines Patienten vorzusehen.

Hierdurch können dieselben Vorteile erreicht werden, wie sie bereits im Zusammenhang mit der erfindungsgemäßen Vorrichtung zur Verminderung der Retraktion der Ränder einer geöffneten Faszie eines Patienten beschrieben wurden.

Eine vorteilhafte Ausgestaltung des Kits sieht vor, dass das Kit zusätzlich ein Abdichtmittel zum Abdichten des offenen Weichteildefekts, insbesondere des Abdomens oder des offenen Rückens, aufweist. Das Abdichtmittel kann als Folie ausgebildet sein, welche auf der dem Körper abgewandten Seite des Schwamms auf den offenen Weichteildefekt, insbesondere die geöffnete Bauchdecke oder die geöffnete Rückenwand, aufgebracht, insbesondere aufgeklebt, werden kann. Durch das Abdichtmittel kann der Körperinnenraum, insbesondere der Bauchraum oder der innerhalb der Rückenwand liegende Raum, flüssigkeitsdicht und gasdicht verschlossen werden. Das Abdichtmittel kann derart angeordnet werden, dass die Vorrichtung zur Verminderung der Retraktion der Ränder einer geöffneten Faszie ganz oder teilweise innerhalb des durch das Abdichtmittel abgedichteten Bereichs liegt. Bevorzugt ist das Abdichtmittel als nicht-klebende, luftundurchlässige Folie ausgebildet. Die nicht-klebende, luftundurchlässige Folie kann unmittelbar an der Vorrichtung zur Verminderung der Retraktion der Faszienränder anliegen. Die nicht-klebende, luftundurchlässige Folie kann über Klebemittel, beispielsweise eine Klebefolie, an dem Körper des Patienten fixiert werden. Das Abdichtmittel weist vorteilhafterweise eine oder mehrere, beispielsweise zwei, Öffnungen auf, durch welche ein Teil der Vorrichtung zur Verminderung der Retraktion der Faszienränder, z. B. eine Aufhängung, hindurch geführt werden kann. Im Bereich der Öffnung kann ein Dichtelement vorgesehen sein, beispielsweise ein ringförmiges und/oder klebendes Dichtelement oder ein Dichtelement nach Art einer Zugvorrichtung, über welche die nicht-klebende, luftundurchlässige Folie in dichtende Anlage mit der Vorrichtung gebracht werden kann.

Bevorzugt weist das Kit eine Vakuumpumpe zur Erzeugung eines Unterdrucks im Körperinnenraum, insbesondere im Bauchraum oder im innerhalb der Rückenwand liegenden Raums, des Patienten auf. Über die Vakuumpumpe können überschüssige Flüssigkeiten und/oder Gase aus dem Körperinnenraum, insbesondere dem Bauchraum oder dem innerhalb der Rückenwand liegenden Raum, abgesaugt werden.

Vorteilhaft ist es, wenn die Vorrichtung zur Verminderung der Retraktion der Ränder der geöffneten Faszie eine außerhalb des Körpers des Patienten anordbare Aufhängung aufweist, an welcher ein mit der Faszie verbindbares Zugorgan anbindbar ist, und das Kit einen Schwamm aufweist, dessen Kontur an die Außenkontur der Aufhängung angepasst ist.

Bei dem Kit können die im Zusammenhang mit der erfindungsgemäßen Vorrichtung beschriebenen Merkmale allein oder in Kombination Verwendung finden.

### Kurze Beschreibung der Zeichnungen

Weitere Vorteile und Einzelheiten der Erfindung sollen nachfolgend anhand der in den Figuren dargestellten Ausführungsbeispiele beschrieben werden. Darin zeigt:
- Fig. 1: eine schematische Schnittdarstellung eines menschlichen Abdomens entlang einer Transversalebene bei geöffnetem Abdomen;
- Fig. 2: eine schematische Schnittdarstellung eines menschlichen Abdomens entlang einer Transversalebene mit einem provisorischen Bauchdeckenverschluss gemäß dem Stand der Technik während der Durchführung einer Unterdruck-Wundtherapie;
- Fig. 3: eine schematische Schnittdarstellung eines menschlichen Abdomens entlang einer Transversalebene mit einer erfindungsgemäßen Vorrichtung zur Verminderung der Retraktion einer geöffneten Faszie gemäß einem ersten Ausführungsbeispiel der Erfindung;
- Fig. 4: eine schematische Schnittdarstellung eines menschlichen Abdomens mit einem erfindungsgemäßen Kit zur Behandlung eines offenen Abdomens mit einer der Vorrichtung nach Fig. 3;
- Fig. 5: eine schematische frontale Darstellung eines offenen Abdomens mit einer Vorrichtung gemäß Fig. 3;
- Fig. 6: eine schematische Schnittdarstellung eines menschlichen Abdomens entlang einer Sagittalebene mit einer Vorrichtung gemäß Fig. 3
- Fig. 7: eine schematische Schnittdarstellung eines menschlichen Abdomens entlang einer Transversalebene mit einer erfindungsgemäßen Vorrichtung zur Verminderung der Retraktion einer geöffneten Faszie gemäß einem zweiten Ausführungsbeispiel der Erfindung;
- Fig. 8: eine schematische Schnittdarstellung eines menschlichen Abdomens mit einem erfindungsgemäßen Kit zur Behandlung eines offenen Abdomens mit einer der Vorrichtung nach Fig. 7
- Fig. 9: eine schematische frontale Darstellung eines offenen Abdomens mit einer Vorrichtung gemäß Fig. 7;
- Fig. 10: eine schematische Schnittdarstellung eines menschlichen Abdomens entlang einer Sagittalebene mit einer Vorrichtung gemäß Fig. 7;
- Fig. 11: eine schematische Schnittdarstellung eines menschlichen Abdomens entlang einer Transversalebene mit einer erfindungsgemäßen Vorrichtung zur Verminderung der Retraktion einer geöffneten Faszie gemäß einem dritten Ausführungsbeispiel der Erfindung;
- Fig. 12: eine schematische Schnittdarstellung eines menschlichen Abdomens entlang einer Transversalebene mit einer erfindungsgemäßen Vorrichtung zur Verminderung der Retraktion einer geöffneten Faszie gemäß einem vierten Ausführungsbeispiel der Erfindung;
- Fig. 13: eine schematische Darstellung einer Aufhängung und
- Fig.14: eine schematische Schnittdarstellung eines menschlichen Abdomens mit einem erfindungsgemäßen Kit zur Behandlung eines offenen Abdomens gemäß einem weiteren Ausführungsbeispiel.
- Fig. 15a: eine schematische Schnittdarstellung einer Vorrichtung zur Verminderung der Retraktion einer geöffneten Faszie gemäß einem fünften Ausführungsbeispiel;
- Fig. 15b: eine schematische Schnittdarstellung der Vorrichtung aus Fig. 15a;
- Fig. 15c: eine schematische Schnittdarstellung eine Abwandlung der Vorrichtung aus Fig. 15a;
- Fig. 16: eine schematische Darstellung eines offenen Rückenwanddefekts mit einer Vorrichtung zur Verminderung der Retraktion einer geöffneten Faszie;
- Fig. 17: eine schematische Schnittdarstellung eines offenen Rückenwanddefekts entlang einer Sagittalebene mit einer Vorrichtung gemäß Fig. 16;
- Fig. 18: eine schematische Schnittdarstellung eines menschlichen Rumpfes entlang der Transversalebene A-A' aus Fig. 17;
- Fig. 19: eine schematische Schnittdarstellung eines menschlichen Rumpfes entlang der Transversalebene B-B' aus Fig. 17
- Fig. 20: eine schematische Schnittdarstellung eines menschlichen Rumpfes entlang der Transversalebene A-A' aus Fig. 17 mit einem erfindungsgemäßen Kit zur Behandlung eines offenen Rückens;
- Fig. 21: eine schematische Schnittdarstellung eines menschlichen Rumpfes entlang der Transversalebene B-B' aus Fig. 17 mit einem erfindungsgemäßen Kit zur Behandlung eines offenen Rückens mit einer der Vorrichtung nach Fig. 16; und
- Fig. 22: eine Abwandlung des in der Fig. 7 gezeigten Ausführungsbeispiels mit einer Umlenkeinrichtung.

### Ausführungsbeispiele der Erfindung

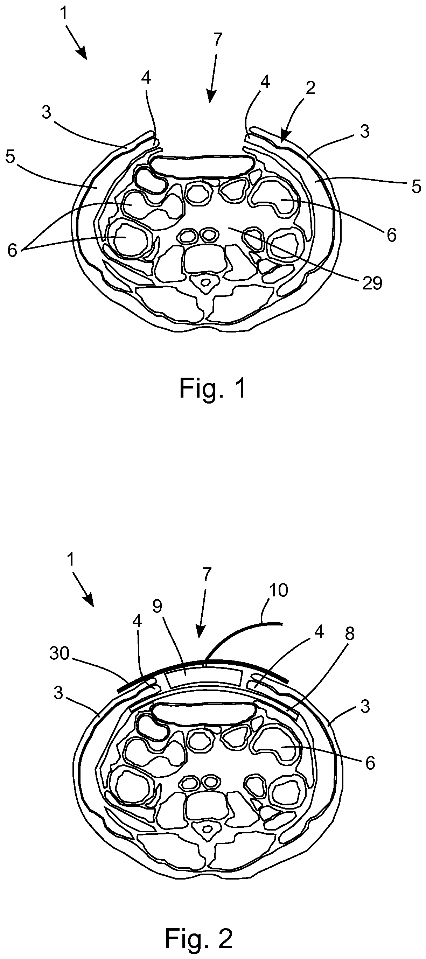

In der **Fig. 1** ist ein transversaler Schnitt durch ein geöffnetes menschliches Abdomen dargestellt. Die Bauchdecke 2 besteht im Wesentlichen aus der Haut 3 und dem unter der Haut 3 liegenden Bauchmuskel 5 sowie den Faszien 4. Zwischen der Haut 3 und dem Bauchmuskel 5 ist in den Figuren nicht dargestelltes Unterhautgewebe angeordnet. Die Faszien 4 sind für die Stabilität der Bauchdecke 2 von hoher Bedeutung. Die Faszien 4 umhüllen den Bauchmuskel 5 und liegen auf der dem Bauchraum 29 zugewandten Seite der Haut 3.

Bei einigen Krankheitsbildern, welche mit einer intraabdominellen Druckerhöhung, einem abdominellen Kompartmentsyndrom, einhergehen, kann es erforderlich werden, den Druck durch eine Eröffnung der Bauchdecke 2 des Patienten 1 zu senken und überschüssige Gase und/oder Flüssigkeiten durch chirurgische Maßnahmen geschaffene Bauchdeckenöffnung 7 aus dem Bauchraum 29 zu entfernen. Hierbei kann es zu einem Heraustreten innenliegender Organe, beispielsweise des Darms 6, durch die Bauchdeckenöffnung 7 kommen.

Um der Entstehung von Infektionen im Bauchraum 29 entgegenzuwirken und die Pflege des offenen Abdomens zu erleichtern, wird die Bauchdeckenöffnung 7 in der Regel provisorisch abgedichtet und gleichzeitig eine Unterdrucktherapie durchgeführt. Wie der Darstellung in **Fig. 2** zu entnehmen ist, wird der Bauchraum 29 dabei beispielsweise über einen unterhalb der Bauchdecke 2 angeordneten Schwamm 8 abgedeckt. Zwischen dem Schwamm 8 und dem Darm 6 wird bevorzugt eine in den Figuren nicht dargestellte Folie oder ein Trennmaterial angeordnet, um ein Austrockenen des Darms 6 zu verhindern. In die Bauchdeckenöffnung 7 wird ein vorzugsweise weiterer Schwamm 9 eingebracht und die Bauchdeckenöffnung 7 wird mit einem Abdichtmittel 30, welches üblicherweise als gasdichte Klebefolie ausgebildet ist, abgedichtet. Der Schwamm 9 wird über einen Saugschlauch 10 mit einer Vakuumpumpe verbunden. Über die Vakuumpumpe wird ein Unterdruck angelegt, so dass durch den Saugschlauch 10 unerwünschte Flüssigkeiten und/oder Gase aus dem Bauchraum 29 entfernt werden können. Diese Unterdrucktherapie wird in der Regel über eine Dauer von einigen Tagen bis hin zu mehreren Wochen durchgeführt. Der Fachmann versteht, dass die Abdeckung der Baudeckenöffnung 7 auch auf andere Art und Weise erfolgen kann.

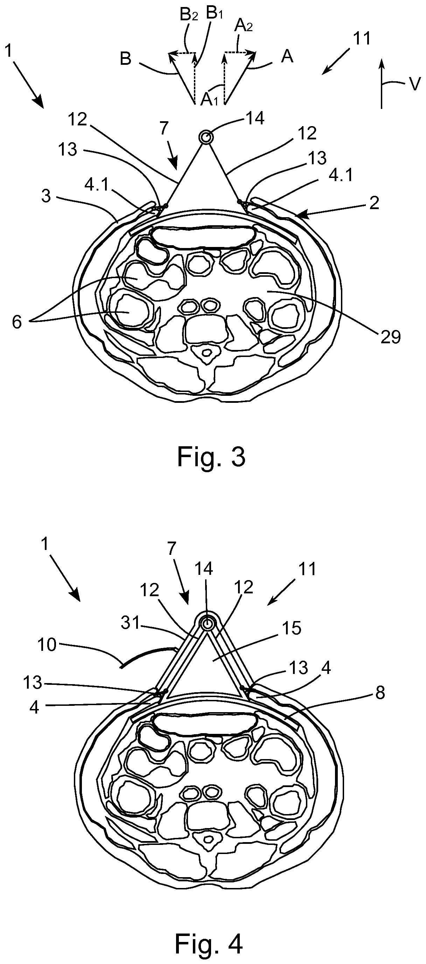

Damit sich die Ränder 4. 1 der Faszie 4 in dieser Zeitspanne nicht übermäßig zurückziehen und das Schließen der Faszie 4, d.h. das Verbinden der Faszienränder 4.1, nach der Periode des offenen Abdomens mit unproblematischer Wundheilung möglich ist, wird nach dem Öffnen der Bauchdecke 2 eine erfindungsgemäße Vorrichtung 11 zur Verminderung der Retraktion der Faszie 4 eingesetzt, vgl. Fig. 3.

Wie der Darstellung in **Fig. 3** zu entnehmen ist, werden über die erfindungsgemäße Vorrichtung 11 Zugkräfte A, B mit einer vom Körper des Patienten 1 weg gerichteten Kraftkomponente A₁, B₁ derart auf die Ränder 4.1 der Faszie 4 aufgebracht, dass die Vorrichtung die Ränder 4.1 auf Spannung und voneinander beabstandet hält. Die Bauchdecke 2, und damit auch die Faszie 4, wird offen gehalten. Die Bauchdeckenöffnung 7 hat eine Ausdehnung von mehr als 1 cm, bevorzugt von mehr als 5 cm, so dass ein ungehinderter Zugang zum Bauchraum 29 möglich ist.

Die Vorrichtung 11 weist vorzugsweise mehrere Zugorgane 12 auf, über welche die Zugkräfte A, B auf die Faszienränder 4.1 eingeleitet werden. Die Zugorgane 12 sind vorzugsweise als Fäden ausgebildet. Alternativ können Drähte, Seile oder Netze verwendet werden. Die Zugorgane 12 können elastisch oder nicht-elastisch ausgebildet sein. Grundsätzlich ist es möglich, die Zugorgane 12 unmittelbar mit der Faszie 4 zu verbinden. Um ein Ausreißen der Zugorgane 12 und eine Schädigung der Faszie 4 zu verhindern, werden die Zugorgane 12 vorzugsweise über ein als Netz ausgebildetes, flächiges Verbindungsmittel 13 mit der Faszie verbunden. Das flächige Verbindungsmittel 13 kann die erheblichen Zugkräfte auf eine größere Fläche verteilen und damit die Einleitung in die Faszie 4 verbessern. Das Netz kann über die gesamte Dauer der Behandlung des offenen Abdomens mit der Faszie 4 verbunden bleiben. Die Zugorgane 12 können hingegen bei Bedarf ausgetauscht werden.

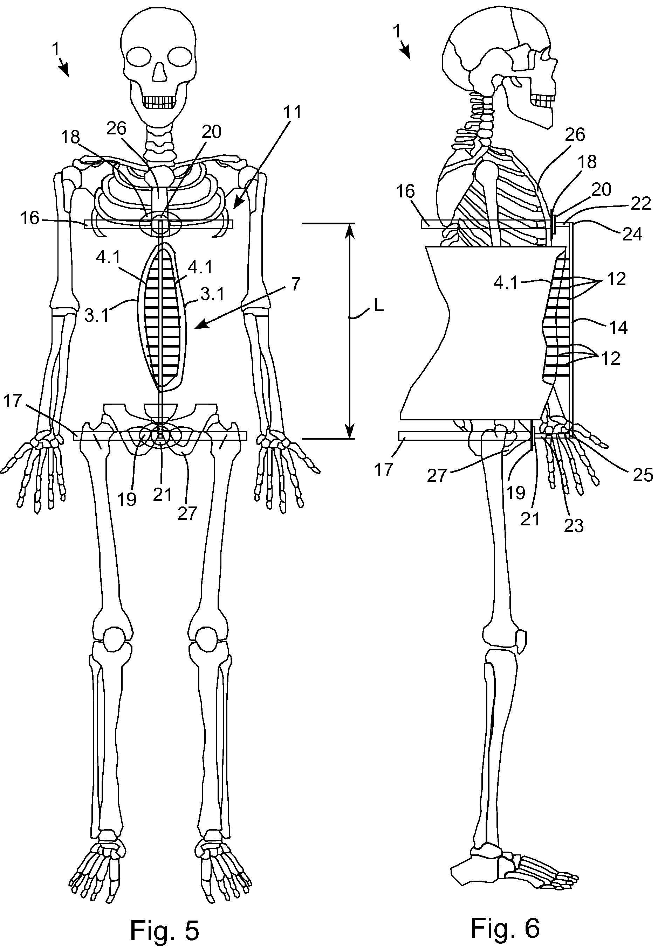

Die Zugorgane 12 sind mit ihren jeweiligen der Faszie 4 abgewandten Seiten mit einer Aufhängung 14 verbunden. Die Aufhängung 14 ist außerhalb des Körpers des Patienten 1 im Bereich vor der geöffneten Bauchdecke 2 angeordnet. Die Aufhängung 14 ist vorzugsweise als mindestens eine Stange ausgebildet, die in vertikaler Richtung entlang der Bauchdeckenöffnung 7 verläuft und sich vorzugsweise in etwa vom Bereich des Brustbeins 26 bis hin zum vorderen Beckenring 27 des Patienten 1 erstreckt, vgl. **Fig. 5** und **Fig. 6****.**

Die Anbindung der Zugorgane 12 an der Aufhängung 14 kann dadurch erfolgen, dass die Zugorgane 12 durch eine in den Figuren nicht dargestellte Durchgangsöffnung in der Aufhängung 14 geführt und dann beispielsweise verknotet werden. Alternativ können die Zugorgane 12 jeweils über eine an der Aufhängung 14 angeordnete Spannvorrichtung an der Aufhängung 14 angebunden sein. Die Spannvorrichtung kann einen Kraftverstärker aufweisen. Eine weitere alternative Anbindung der Zugorgane 12 an der Aufhängung 14 kann über an der Aufhängung 14 angeordnete Rillen erfolgen. Die Zugorgane 12 können vorteilhafterweise durch die Rillen geführt werden.

Wie den Darstellungen in Fig. 3-6 ferner entnommen werden kann, können sämtliche Zugorgane 12 über Verbindungsstellen an der Aufhängung 14 angebunden werden, die entlang einer gemeinsamen Gerade angeordnet sind, wodurch ein kompakter Aufbau ermöglicht wird. Alternativ kann eine Aufhängung mit gekrümmten Verlauf verwendet werden, so dass die Verbindungsstellen auf einer gekrümmten Linie, beispielsweise einer Kreislinie oder der Umfangslinie einer geschlossenen, gekrümmten Linie, z. B. einer Ellipse angeordnet sind.

Die Aufhängung 14 ist vorzugsweise über zwei Befestigungseinrichtungen 16, 17 am Körper des Patienten 1 festgelegt. Die Befestigungseinrichtungen 16, 17 sind als Gurtbefestigungen ausgebildet, die im Bereich des Brustbeins 26 und im Bereich des vorderen Beckenrings 27 fest um den Körper des Patienten 1 geschnallt sind, so dass ein Verrutschen der Befestigungseinrichtungen 16, 17 möglichst verhindert wird. Die im Bereich des vorderen Beckenrings 27 angeordnete Befestigungseinrichtung 17 ist bevorzugt derart ausgebildet sein, dass bei angelegter Befestigungseinrichtung 17 die Durchführung einer Analhygiene möglich ist. Ventral weisen die Befestigungseinrichtungen 16, 17 hier jeweils ein Druckverteilungsmittel, beispielsweise eine als Gelplatte 18, 19 ausgebildete Abstützung auf, über welche sich die Aufhängung 14 auf dem Körper des Patienten 1 abstützt. Optional kann das Druckverteilungsmittel mehrere, insbesondere zwei, drei, vier, fünf oder sechs, Kammern aufweisen, die wechselweise mit einem Medium, wie Wasser. Luft oder Gel gefüllt werden können. An den Abstützungen 18, 19 sind jeweils Halteplatten 20, 21 und eine sich in ventraler Richtung erstreckende Stütze 22, 23 angeordnet. Über die Abstützungen 18, 19, die Halteplatten 20, 21 und die Stützen 22, 23 sind die Befestigungseinrichtungen 16, 17 mit den beiden Enden der Aufhängung 14 verbunden.

Die Befestigungseinrichtungen 16, 17, die Stützen 22, 23 sowie die Aufhängung 14 sind derart ausgebildet, dass die an den Körperbau des Patienten 1 angepasst werden können. Die Befestigungseinrichtungen 16, 17 können beispielsweise längenverstellbar sein, so dass diese an den Körperumfang des jeweiligen Patienten 1 angepasst werden können. Die Stützen 22, 23 sind beispielsweise teleskopierbar ausgebildet, beispielsweise als Teleskopstützen, so dass der Abstand der beiden Enden der Aufhängung 14 von der Bauchdecke 2 individuell eingestellt werden kann. Ferner ist die Aufhängung 14 insbesondere teleskopierbar ausgebildet, so dass die Länge der Aufhängung 14 in einer vertikal, also in einer Richtung parallel zu einer Verbindungslinie vom Scheitel zur Sohle, entlang des Abdomens verlaufenden Richtung eingestellt werden kann. Alternativ können die Stützen 22, 23 verschiebbar an der Aufhängung 14 angebunden sein, so dass der Abstand der Stützen 22, 23 voneinander an die Größe der Bauchdeckenöffnung 7 angepasst werden kann.

Bevorzugt sind zwischen den Stellen, an denen die Zugorgane 12 an die Aufhängung 14 angebunden sind, Federmittel vorgesehen. Bei einer teleskopierbar ausgebildeten Aufhängung 14, beispielsweise einem Teleskopstab, sind bevorzugt zwischen den Stellen, an denen die Zugorgane 12 an die Aufhängung 14 angebunden sind, federnde Teleskopelemente angeordnet.

Die Stützen 22, 23 sind mit der Aufhängung 14 jeweils über ein Gelenk 24, 25 gekoppelt, welches als Kugelgelenk oder Scharnier ausgebildet sein kann. Insofern ist die Aufhängung 14 gegenüber den Stützen 22, 23 und der jeweiligen mit den Stützen verbundenen Befestigungseinrichtung 16, 17 beweglich gelagert, so dass Bewegungen des Patienten 1 beim Atmen oder Husten ausgeglichen werden können. Alternativ ist es möglich, dass wahlweise nur die Kopplung zwischen der oberen Befestigungseinrichtung 16 und der Aufhängung 14 oder nur die Kopplung zwischen der unteren Befestigungseinrichtung 17 und der Aufhängung 14 über ein Gelenk 24 erfolgt und die jeweilige andere Kopplung von Befestigungseinrichtung 16 oder 17 und Aufhängung 14 starr ausgebildet ist. Weiter alternativ können die Befestigungseinrichtungen 16, 17 und die Aufhängung 14 starr gekoppelt sein. Eine weitere Alternative sieht vor, dass die Stützen 22, 23 jeweils über ein Gelenk mit einer Befestigungseinrichtung 16, 17 oder mit einer Halteplatte 20, 21 gekoppelt sind.

Wie in Fig. 3 gezeigt, werden über die Vorrichtung 11 Zugkräfte A, B mit einer vom Körper weg gerichteten Kraftkomponente A₁, B₁ erzeugt. Die Zugkräfte A, B verlaufen im Wesentlichen in ventrolateraler Richtung, d.h. sie sind vom Patienten seitlich nach vorn gerichtet. Die Faszienränder 4.1 werden dadurch in einem Abstand voneinander gehalten, der mehr als 1 cm, bevorzugt mehr als 5 cm betragen kann. Gleichzeitig werden die Faszienränder 4.1 in ventraler Richtung V, also in einer Richtung senkrecht zur gedachten Verbindungslinie der Faszienränder 4.1, gespannt und davon abgehalten, sich nach seitlich zu retrahieren. Im Gegensatz zu Vorrichtungen, welche lediglich eine Kraftkomponente A₂, B₂ in Richtung der Bauchdeckenöffnung 7 erzeugen, kann bei der erfindungsgemäßen Vorrichtung eine Behandlung am offenen Abdomen erfolgen. Der Bauchraum 29 wird insofern durch die Bauchdeckenöffnung 7 und durch die Ventralverlagerung der Bauchdecke 2 vergrößert.

Wenn die Vorrichtung 11 an dem Körper des Patienten 1 angeordnet ist, kann die Bauchdeckenöffnung durch Tücher abgedeckt werden. Alternativ kann eine Unterdruck-Wundtherapie durchgeführt werden, wie nachfolgend näher erläutert werden soll.

Die Bestandteile eines für eine Behandlung des offenen Abdomens geeigneten Kits sind in der **Fig. 4** dargestellt. Das Kit weist neben der vorstehend beschriebenen Vorrichtung 11 zur Verminderung der Retraktion der Ränder 4.1 einer geöffneten Faszie 4 einen Schwamm 15 auf, welcher die Bauchdeckenöffnung 7 ausfüllt. Der Schwamm 15 weist vorzugsweise einen im Wesentlichen dreieckigen Querschnitt auf, so dass er den durch die Zugorgane 12 begrenzten Bereich zwischen den Rädern 4.1 der Faszie und der Aufhängung 14 ausfüllt. Der Schwamm 15 ist bevorzugt formstabil ausgebildet. Besonders bevorzugt weist der Schwamm 15 einen innenliegenden Bereich mit gegenüber seiner Oberfläche erhöhter Festigkeit oder eine innenliegende, nicht deformierbare Grundform auf. Die Zugorgane 12 und die Aufhängung 14 können insbesondere durch einen weiteren Schwamm 31 abgedeckt sein. Der Schwamm 31 ist bevorzugt mit den Zugorganen 12 verbindbar ausgestaltet. Der Schwamm 31 kann einen Abschnitt aufweisen, dessen Form an die Außenkontur der Aufhängung 14 angepasst ist. An der außenliegenden Oberfläche des Schwamms 31 kann bevorzugt ein in den Figuren nicht dargestelltes gas- und/oder flüssigkeitsdichtes Abdichtmittel 30 angeordnet werden, z. B. eine Klebefolie oder eine gasdichte Hülle. An den Schwamm 31 kann ein Saugschlauch 10 angeschlossen werden, so dass eine vorstehend im Zusammenhang mit Fig. 2 beschriebene Unterdrucktherapie durchgeführt werden kann.

Gemäß einer Abwandlung des in der Fig. 3-6 gezeigten Ausführungsbeispiels können die Zugorgane 12 bevorzugt Federmittel aufweisen. Über das Federmittel kann das jeweilige Zugorgan 12 in Richtung der Zugkraft A, B vorgespannt werden und damit auf Spannung gehalten werden.

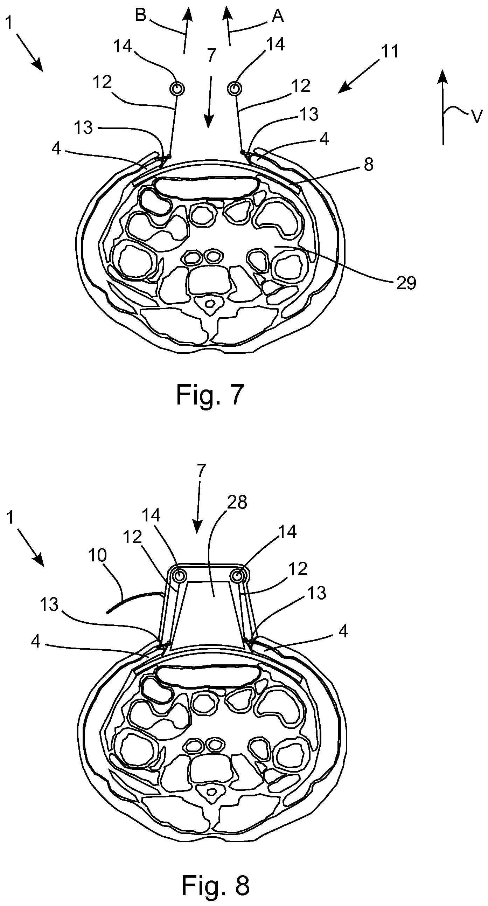

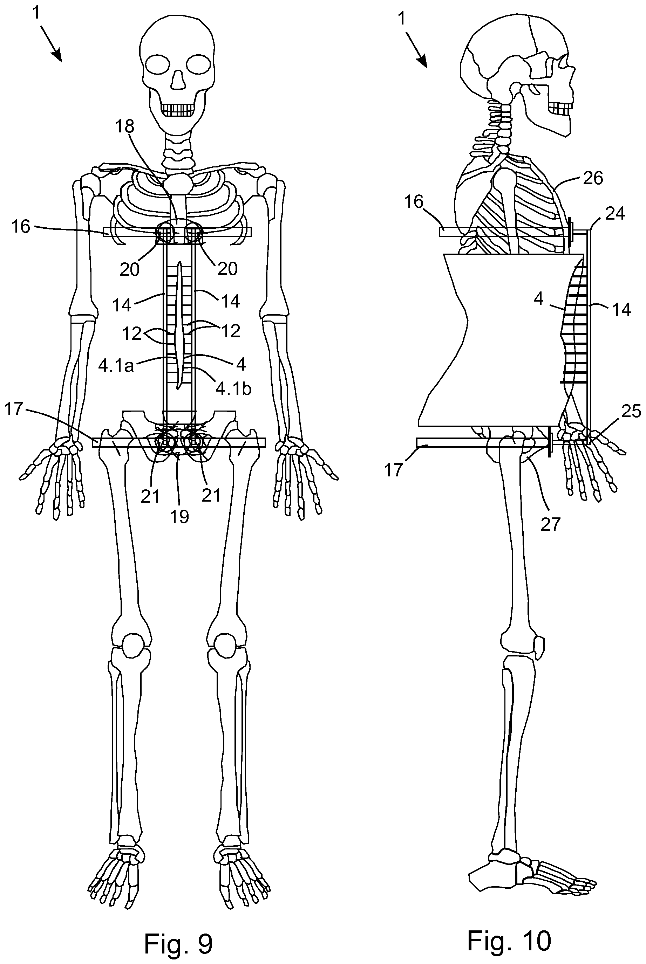

Das in der **Fig. 7-10** gezeigte, zweite Ausführungsbeispiel einer Vorrichtung 11 zur Verminderung der Retraktion der Faszienränder 4.1 gleicht im Wesentlichen dem in den Fig. 3-6 gezeigte ersten Ausführungsbeispiel. Im Unterschied zu dem ersten Ausführungsbeispiel sind bei der Vorrichtung 11 nach Fig. 7-10 mehrere Aufhängungen 14 vorgesehen. Die mit einem ersten Rand 4.1a der Faszie 4 verbundenen Zugorgane 12 sind mit einer ersten Aufhängung 14 und die mit einem zweiten Rand 4.1b der Faszie 4 verbundenen Zugorgane 12 sind mit einer zweiten Aufhängung 14 verbunden. Die Aufhängungen 14 sind durch einen Abstand voneinander getrennt, welcher im Wesentlichen dem Abstand der beiden Faszienränder 4.1a, 4.1b entspricht. Die Zugkräfte A, B greifen an der Faszie 4 in einer im Wesentlichen ventralen Richtung V an, d.h. die Zugkraft ist vom Patienten 1 aus betrachtet nach vorn gerichtet. Gemäß dem zweiten Ausführungsbeispiel sind die beiden Aufhängungen 14 parallel angeordnet. Alternativ können diese quer zueinander angeordnet sein. Durch die Verwendung mehrerer Aufhängungen 14 wird im dem der Bauchdeckenöffnung 7 vorgelagerten Bereich ein Freiraum gebildet, durch welchen der Zugang zu dem offenen Abdomen erleichtert wird, vgl. Fig. 7 und Fig. 9.

Bei der Behandlung des offenen Abdomens im Rahmen einer Unterdrucktherapie kann ein Kit mit einem in die Bauchdeckenöffnung 7 eingebrachten Schwamm 28 zur Anwendung kommen, der bevorzugt einen rechteckigen oder trapezförmigen Querschnitt aufweist, vgl. Fig. 8. Ansonsten kann das Kit wie das anhand der Fig. 4 erläuterte Kit ausgebildet sein.

In Abwandlung des zweiten Ausführungsbeispiels können die Aufhängungen 14 Abschnitte eines Rahmens sein, welcher der Bauchdeckenöffnung 7 vorgelagert anordbar ist. Der Rahmen kann mehrere insbesondere parallel angeordnete Aufhängungen aufweisen, die in vertikaler Richtung, also parallel zu einer gedachten Linie vom Scheitel zur Sohle des Patienten 1, entlang des Abdomens angeordnet werden können. Der Rahmen kann bevorzugt mehrere Abschnitte aufweisen, die gegeneinander bewegbar sind. Beispielsweise können die Abschnitte des Rahmens über Gelenke miteinander verbunden sein.

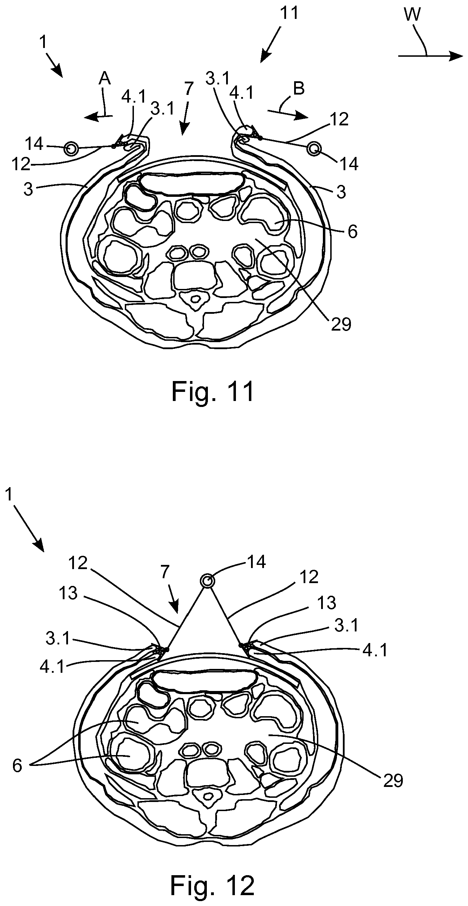

In **Fig. 11** ist ein drittes Ausführungsbeispiel der Erfindung dargestellt. Im Unterschied zu dem zweiten Ausführungsbeispiel ist der Abstand zwischen den Aufhängungen 14 größer als der Abstand zwischen den Rändern 4.1 der Faszie 4. Bei der Vorrichtung 11 gemäß dem dritten Ausführungsbeispiel werden auf die Faszienränder 4.1 insbesondere Zugkräfte A, B aufgebracht, die eine vom Körper weg gerichtete Kraftkomponente aufweisen, welche parallel zu einer gedachten Verbindungslinie zwischen den beabstandeten Faszienrändern 4.1 verläuft. Die Zugkräfte A, B sind insbesondere im Wesentlichen parallel zur Bauchdecke 2 des Patienten 1 aber von der Bauchdeckenöffnung 7 weg gerichtet. Die Zugkräfte A, B greifen im Wesentlichen in lateraler Richtung W an der Fasize 4 an. Die Zugkräfte A, B greifen bevorzugt in einer von der Mitte der Bauchdeckenöffnung 7 weg gerichteten, parallel zu der gedachten Verbindungslinie der Faszienränder 4.1 verlaufenden Richtung an der Faszie 4 an. Die Faszienränder 4.1 sind insbesondere um die Hautränder 3.1 umgeschlagen.

In **Fig. 12** ist ein viertes Ausführungsbeispiel der Erfindung dargestellt. Im Unterschied zu dem ersten Ausführungsbeispiel ist das Verbindungsmittel 13 nicht nur mit den Faszienrändern 4.1 sondern auch mit den Hauträndern 3.1 verbunden. Durch die Vorrichtung 11 nach Fig. 12 kann eine Zugkraft A, B mit einer vom Körper des Patienten 1 weg gerichteten Kraftkomponente derart auf die Ränder 4.1, 3.1 von Faszie 4 und Haut 3 aufgebracht werden, dass die Vorrichtung 11 die Ränder 4.1, 3.1 von Faszie 4 und Haut 3 auf Spannung und voneinander beabstandet hält und dass weiterhin eine geöffnete Bauchdecke 2 besteht.

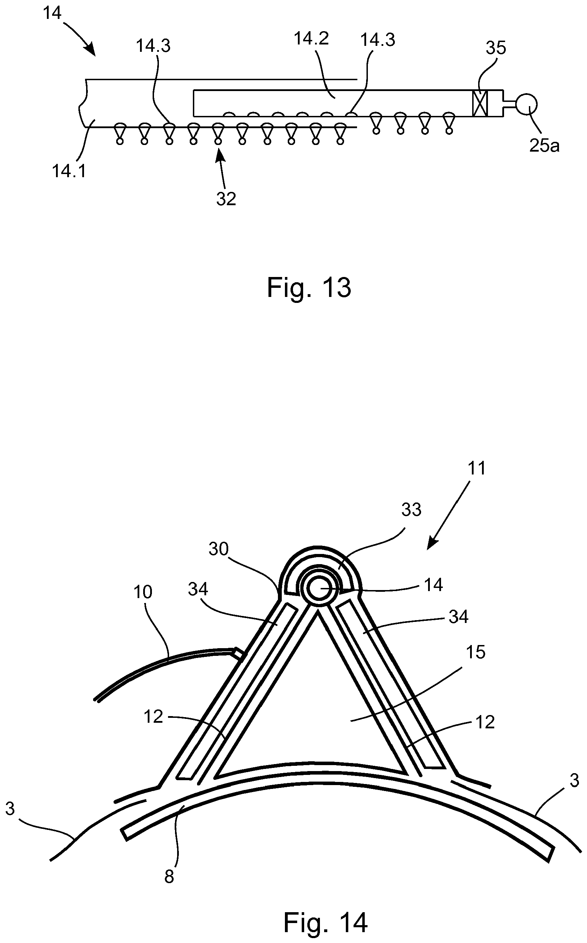

**Fig. 13** zeigt ein Beispiel einer Aufhängung 14, wie sie in einer Vorrichtung 11 gemäß einem der vorstehend beschrieben Ausführungsbeispiele verwendet werden kann. Die Aufhängung 14 ist als Teleskopstange ausgebildet und weist zumindest zwei gegeneinander bewegbare Teilstücke 14.1 und 14.2 auf. Ein erstes Teilstück 14.1 weist einen größeren Querschnitt als ein zweites Teilstück 14.2 auf, so dass das zweite Teilstück 14.2 in das erste Teilstück 14.1 eingeschoben werden kann. An dem zweiten Teilstück kann ein Gelenkkopf 25a eines Kugelgelenks angeformt oder angebunden sein.

Die Aufhängung 14, insbesondere die Teilstücke 14.1, 14.2, weisen mehrere Verbindungsstellen 14.3 auf, über welche die Zugorgane 12 angebunden werden können. Ferner können Verbinder 32 vorgesehen sein, welche derart ausgebildet sind, dass sie an den Verbindungsstellen 14.3 reversibel angebunden werden können. Die Verbindungsstellen 14.3 sind bevorzugt als Öffnungen ausgebildet. Die Verbinder 32 sind bevorzugt derart ausgebildet, dass sie in die Öffnungen einsteckbar sind.

Im Bereich zwischen den Verbindungsstellen 14.3 und dem mit der Abstützung 18, 19 verbundenen Ende der Aufhängung 14, insbesondere dem Ende der Teilstücke 14.1, 14.2, ist optional ein elastisches Element 35, beispielsweise eine Feder, angeordnet. Durch das elastische Element 35 können Bewegungen der mit der Abstützung 18, 19 verbundenen Enden der Aufhängung 14, insbesondere durch Atembewegungen oder Husten des Patienten, ausgefedert werden. In einer Abwandlung des Ausführungsbeispiels gemäß Fig. 13 kann ein elastisches Element 35 im Bereich zwischen zwei Verbindungsstellen 14.3 angeordnet sein.

In der Darstellung in Fig. 13 sind zwei Teilstücke 14.1 und 14.2 gezeigt. Es ist möglich, die Aufhängung 14 derart auszubilden, dass sie mindestens drei Teilstücke aufweist, wobei das in der Fig. 13 gezeigte linke Teilstück 14.1 ein Mittelstück mit einem größeren Querschnitt bildet, in welches die beiden anderen Teilstücke 14.2 mit einem kleineren Querschnitt eingeschoben werden können. An dem Mittelstück 14.1 kann eine Abdichtung vorgesehen sein, so dass die Länge der Aufhängung 14 durch Bewegung der äußeren Teilstücke 14.2 gegenüber dem Mittelstück 14.1 verändert werden kann, wobei das geöffnete Abdomen abgedichtet ist. Die Abdichtung kann beispielsweise nach Art eines Dichtrings ausgebildet sein, welcher das Mittelstück 14.1 abdichtend umschließt. Die äußeren Teilstücke 14.2 können sich in einem Bereich befinden, der nicht in die Abdichtung des Bauchraums - den Versiegelungsbereich - einbezogen ist. Die äußeren Teilstücke 14.2 können z.B. vor dem Schambereich des Patienten angeordnet werden. Der Schambereich wird bei der Abdichtung des Bauchraums nicht in den Versiegelungsbereich mit einbezogen, da dort die Anbringung von Dichtelementen erschwert ist.

In einer Abwandlung dieses Ausführungsbeispiels kann eine einstückig ausgebildete Aufhängung 14, insbesondere nach Art einer Stange, vorgesehen sein, wobei an der Aufhängung 14 eine oder mehrere Abdichtungen vorgesehen sind. Die Abdichtungen können nach Art von Dichtringen ausgestaltet sein, welche die Aufhängung 14, insbesondere die Stange, dichtend umschließen.

Die Darstellung in **Fig. 14** zeigt eine Weiterbildung des in der Fig. 4 gezeigten Kits. Das Kit nach Fig.14 weist im Unterschied zu dem Kit nach Fig. 4 zusätzlich einen Abdeckschwamm 33 auf, dessen Kontur an die Außenkontur der Aufhängung 14 angepasst ist. Zudem sind zwei Seitenschwämme 34 vorgesehen, die außen auf den Schwamm 15 und die Zugorgane 12 aufgelegt werden können.

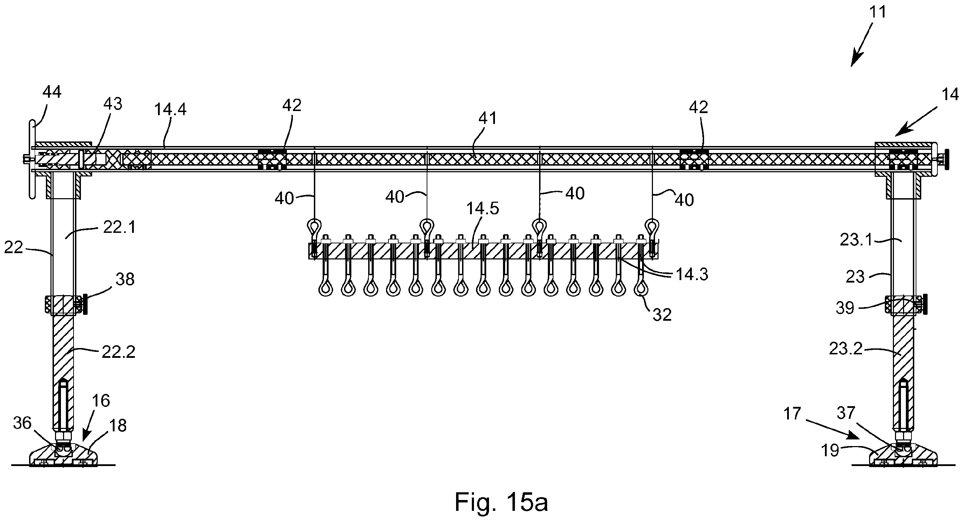

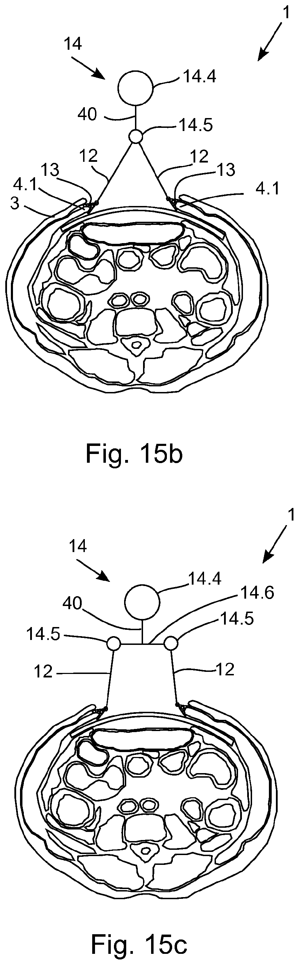

In der **Fig. 15a** und **Fig. 15b** ist ein weiteres Ausführungsbeispiel einer Vorrichtung 11 zur Verhinderung der Retraktion der Faszien dargestellt. Die Vorrichtung 11 weist eine Aufhängung 14 auf, über welche beispielsweise als Fäden ausgebildete Zugorgane 12 angebunden werden können. Die Aufhängung 14 ist starr mit zwei Stützen 22, 23 verbunden, über welche die Aufhängung 14 in einer vom Körper des Patienten 1 beabstandeten Position gehalten werden kann. Die Stützen 22, 23 sind als Teleskopstützen ausgebildet, so dass es möglich ist, die Position der Aufhängung 14 über die Stützen 22, 23 einzustellen. Ferner sind die Stützen 22, 23 entlang der Aufhängung 14 verschiebbar und können an verschiedenen Stellen der Aufhängung 14 wahlweise festgelegt werden. Insofern ist es möglich, den Abstand der Stützen 22, 23 an die Größe des Weichteildefekts, beispielsweise die Größe der Bauchdeckenöffnung, anzupassen. Die Stützen 22, 23 verbinden die Aufhängung 14 jeweils mit einer Befestigungseinrichtung 16, 17, die auf der Haut 3 des Patienten 1 aufliegt.

Die Stützen 22, 23 weisen jeweils zwei gegeneinander bewegbare Stützenelemente 22.1, 22.2, 23.1, 23.2 auf. An den Stützen 22, 23 ist jeweils eine Feststellvorrichtung 38, 39 vorgesehen, über welche die Stützenelemente 22.1, 22.2, 23.1, 23.2 gegeneinander festlegbar sind. Die Feststellvorrichtung 38, 39 kann beispielsweise nach Art einer Feststellschraube ausgebildet sein.

Die Stützen 22, 23 weisen bevorzugt eine in den Figuren nicht gezeigte Kraftmesseinrichtung auf, über welche die Summe der durch die Zugorgane aufgebrachten Zugkraft messbar ist. Alternativ kann eine Kraftmesseinrichtung als Teil der Befestigungseinrichtung 16, 17 vorgesehen sein.

Die Vorrichtung 11 ist derart ausgebildet, dass die Zugkraft an der Faszie 4 in einer Richtung angreift, die mit einer virtuellen Verbindungslinie zwischen den Rändern 4.1 der geöffneten Faszie 4 einen Winkel einschließt, der im Bereich von 5° bis 90° oder im Bereich von 10° bis 80° oder im Bereich von 15° bis 70 ° liegt. Bevorzugt liegt der Winkel im Bereich von 30° bis 60°, besonders bevorzugt im Bereich von 35° bis 55°. Beispielsweise kann der Winkel im Wesentlichen 45° betragen. In Bezug auf die Längsachse einer Stütze 22, 23 greift die Zugkraft an der Faszie 4 in einer Richtung an, die mit der Längsachse der Stütze 22, 23 einen Winkel einschließt, der im Bereich von 0° bis 85° oder im Bereich von 10° bis 80° oder im Bereich von 20° bis 75° liegt. Bevorzugt liegt der Winkel im Bereich von 30° bis 60°, besonders bevorzugt im Bereich von 35° bis 55°. Beispielsweise kann der Winkel im Wesentlichen 45° betragen.

An einer der Aufhängung 14 abgewandten Seite weisen die Stützen 22, 23 jeweils ein Gelenk 36, 37 auf, über welches die Stützen 22, 23 mit einer Abstützung 18, 19 einer Befestigungseinrichtung 16, 17 verbunden sind. Die Befestigungseinrichtung 16, 17 kann ferner einen oder mehrere Gurte zur Festlegung der Abstützungen 18, 19 am Patienten 1 aufweisen. Das Gelenk 36, 37 ist bevorzugt als Kugelgelenk ausgebildet. Wenn die Vorrichtung 11 am Körper eines Patienten 1 angeordnet ist, stützen sich die Abstützungen 18, 19 der Befestigungseinrichtungen 16, 17 auf der Haut des Patienten 1 ab, bevorzugt in einem Bereich der Haut, in welchem durch die Vorrichtung 11 keine Zugkräfte auf die unter der Haut liegende Faszie aufgebracht werden.

Alternativ können die Befestigungseinrichtungen 16, 17 einen Verbindungsbereich aufweisen, über welchen die Befestigungseinrichtungen 16, 17 an einem Knochen des Patienten 1 befestigt werden können.

Die Aufhängung 14 der Vorrichtung 11 weist einen zweiteiligen Aufbau auf, der nachfolgend näher beschrieben werden soll. Die Aufhängung 14 weist ein insbesondere starr mit den Stützen 22, 23 verbundenes Halteelement 14.4 und ein gegenüber dem Halteelement 14.4 bewegbares Anbindungselement 14.5 auf. An dem Anbindungselement 14.5 sind mehrere Verbindungsstellen 14.3 vorgesehen, über welche Zugorgane 12 angebunden werden können. Die Verbindungsstellen 14.3 sind beispielsweise als Bohrungen ausgebildet. In den Verbindungsstellen 14.3 sind, insbesondere als Ösenschrauben ausgebildete, Verbinder 32 angeordnet. Alternativ können die Verbinder als in die Verbindungsstellen 14.3 einsteckbare und in den Verbindungsstellen verrastbare Rastelemente mit Ösen ausgebildet sein. Die Position der Verbinder 32 gegenüber dem Anbindungselement 14.5 ist einstellbar, so dass über die Einstellung der Position der Verbinder 32 die Spannung der Zugorgane 12 unabhängig voneinander eingestellt werden kann. Insofern bilden die Verbinder 32 eine Spannvorrichtung, über welche die Spannung der Zugorgane 12 individuell einstellbar ist. Das Anbindungselement 14.5 ist über mehrere Seile 40 oder Ketten mit dem Halteelement 14.4 verbunden, wobei ein erstes Ende eines Seils 40 oder eine Kette fest an dem Anbindungselement 14.5 befestigt ist. Ein zweites Ende des Seils 40 oder der Kette ist derart mit einer in dem Halteelement 14.4 gelagerten Welle 41 verbunden, dass das Seil 40 oder die Kette bei einer Drehung der Welle 41 aufwickelbar ist. Durch das Drehen der Welle 41 kann die Position des Anbindungselements 14.5 und damit die Spannung sämtlicher an dem Anbindungselement 14.5 angebundener Zugorgane 12 gemeinsam eingestellt werden. Insofern wird durch das Drehen der Welle 41 eine gruppenweise Einstellung der Spannung der Zugorgane 12 bzw. die Einstellung des Abstands zwischen dem Halteelement 14.4 und dem Anbindungselement 14.5 ermöglicht. Die Welle 41 ist über eine Feststellkupplung 43 mit einem Betätigungselement 44 verbunden. Die Feststellkupplung 43 ist derart ausgebildet, dass sie eine Drehung der Welle 41 im Regelfall blockiert und nur dann eine Drehung der Welle 41 ermöglicht, wenn das Betätigungselement 44 in Richtung der Drehachse der Welle 41 aus der Aufhängung 14, insbesondere aus dem Halteelement 14.4 gezogen wird.

Das Anbindungselement 14.5 ist entweder getrennt von oder zusammen mit der Welle 41 oder einem Teil der Welle 41 auswechselbar. Die Verbinder 32 können zusammen mit dem Anbindungselement 14.5 ausgewechselt werden. Insofern wird ein Wegwerfanbindungselement bereitgestellt.

Wenn die Vorrichtung 11 im Rahmen einer Unterdruck-Wundtherapie verwendet wird, kann die Aufhängung 14 der Vorrichtung 11 derart angeordnet werden, dass sich die Aufhängung 14 im gasdicht abgedichteten Bereich befindet. Um ein Nachspannen der Zugorgane 12 auch bei angelegtem Unterdruck-Wundverband zu ermöglichen, kann die Aufhängung derart angeordnet werden, dass das Betätigungselement 42 außerhalb des gasdicht abgedichteten Bereichs angeordnet ist. Um ein Eindringen von Luft durch die Aufhängung 14 in den gasdicht abgedichteten Bereich zu verhindern, weist die Aufhängung 14 bevorzugt ein Gehäuse auf, welches gasdicht abgedichtet ist. Beispielsweise kann das Halteelement 14.4 eine oder mehrere Dichtungen 42 aufweisen, welche die Welle 42 gegenüber dem Halteelement 14.4 abdichten. Zur Abdichtung wird eine nicht-klebende, luftundurchlässige Folie verwendet. Die Folie weist zwei Öffnungen auf, durch welche die Aufhängung 14, insbesondere das Haltelement 14.4, geführt ist. Die Folie ist über Dichtmittle gegenüber der Aufhängung 14, insbesondere dem Haltelement 14.4, abgedichtet.

Die **Fig. 15c** zeigt eine Abwandlung des in Fig. 15a und Fig. 15b gezeigten Ausführungsbeispiels, bei welchem die Aufhängung 14 ein Halteelement 14.4 und mehrere, insbesondere zwei, gegenüber dem Halteelement 14.4 bewegbare Anbindungselemente 14.5 aufweist. Die Anbindungselemente 14.5 sind über ein Distanzelement 14.6 miteinander verbunden. Der Abstand der Anbindungselemente 14.5 zueinander ist einstellbar. Zur Abstandseinstellung kann das Distanzelement 14.6 teleskopierbar ausgebildet sein.

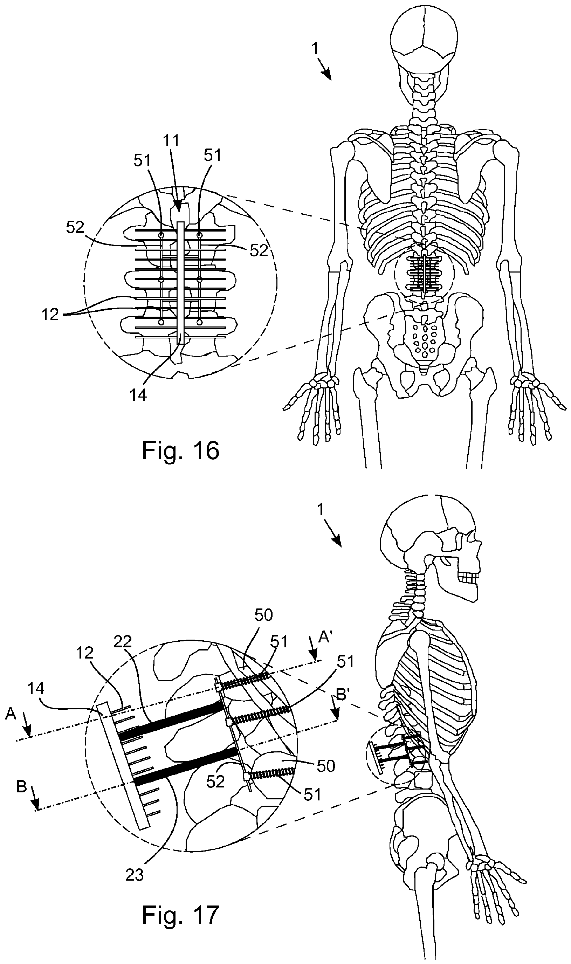

Anhand der Darstellungen in **Fig. 16-21** soll im Folgenden ein Ausführungsbeispiel einer erfindungsgemäßen Vorrichtung 11 beschrieben werden, welches zur Behandlung einer offenen Rückenwand geeignet ist. Die Eröffnung der Rückenwand kann beispielsweise zur Behandlung von Wirbelbrüchen, von Wirbelgleiten, von Bandscheibenschäden, von Verengungen des Rückenmarkkanals, von Infektionen oder Tumoren an der Wirbelsäule sowie von Fehlbildungen und Fehlstellungen der Wirbelsäule erforderlich werden. Insbesondere bei auftretenden Infektionen nach stattgehabter Operation kann mitunter die Rückenwand aus Gründen der Infektbehandlung offen gelassen werden. Um der Entstehung von Infektionen entgegenzuwirken und die Pflege der offenen Rückenwand zu erleichtern, wird der Rückenwanddefekt in der Regel provisorisch abgedichtet. Optional kann eine Unterdruck-Wundtherapie durchgeführt werden.

Im Bereich des Rückens umhüllt eine Faszie 4 den unter der Haut 3 liegenden Rückenmuskel 56.

Die Vorrichtung 11 zur Verhinderung der Retraktion der Faszien 4 weist eine nach Art einer Stange ausgebildete Aufhängung 14 auf, die mit Stützen 22, 23 verbunden ist. Die Stützen 22, 23 verbinden die Aufhängung 14 mit einer Befestigungseinrichtung, welche als bereits im Körper des Patienten angeordnetes Implantat, also als Fixateur interne, ausgebildet ist. Das Implantat besteht aus mehreren, insbesondere zwei, Längsstäben 52, die über nicht in den Figuren gezeigte Madenschrauben an den Pedikelschrauben 51 festgelegt sind. Die Pedikelschrauben 51 durchdringen den Pedikel des Wirbels 50 und erstrecken sich bis in den Bereich des Wirbelkörpers des Wirbels 50. Das Implantat weist mehrere Längsstangen 52 auf, welche jeweils mit den Pedikelschrauben 51 in zwei oder mehreren Wirbeln 50 verbunden sind und die Stellung dieser Wirbel 50 zueinander fixieren.

An der Aufhängung 14 können Zugorgane 12 angebunden werden, über welche Zugkräfte auf die Faszienränder 4.1 aufgebracht werden können. Diese Zugorgane 12 sind bevorzugt als Fäden, Drähte, Seile oder Netze ausgebildet. Die Zugorgane 12 können elastisch oder nicht-elastisch ausgebildet sein. Grundsätzlich ist es möglich, die Zugorgane 12 unmittelbar mit der Faszie 4 zu verbinden. Um ein Ausreißen der Zugorgane 12 und eine Schädigung der Faszie 4 zu verhindern, werden die Zugorgane 12 vorzugsweise über ein als Netz ausgebildetes, flächiges Verbindungsmittel 13 mit der Faszie verbunden.

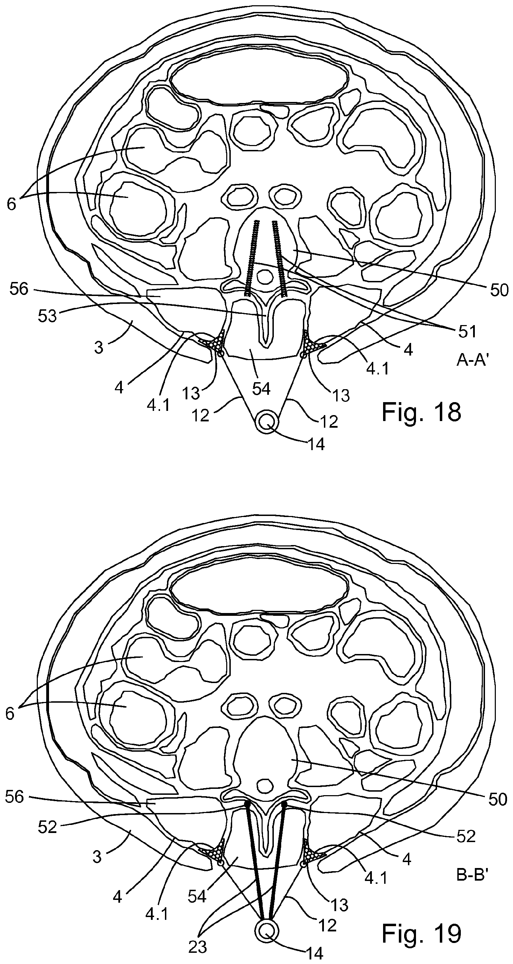

Wie den Darstellungen in Fig. 18-21 entnommen werden kann, ist die Aufhängung 14 außerhalb des Körpers des Patienten 1 mit einem gewissen Abstand von dem offenen Defekt in der Haut 3 angeordnet. In einer Abwandlung des Ausführungsbeispiels ist es möglich, dass die Aufhängung 14 in der Ebene des Hautdefekts oder innenliegend der Ebene des Hautdefekts aber außerhalb der Ebene des Fasziendefekts angeordnet ist. In weiterer Abwandlung kann die Vorrichtung gemäß diesem Ausführungsbeispiel mehrere Aufhängungen 14 aufweisen. Beispielsweise ist es möglich, dass die Zugorgane 12 an mehreren Aufhängungen 14 angebunden sind, die als im Wesentlichen parallel verlaufende Stangen ausgebildet sind, wie es vorstehend im Zusammenhang mit den Darstellungen in Fig. 7 und 8 für die Anwendung im Bereich des Abdomens beschrieben ist.

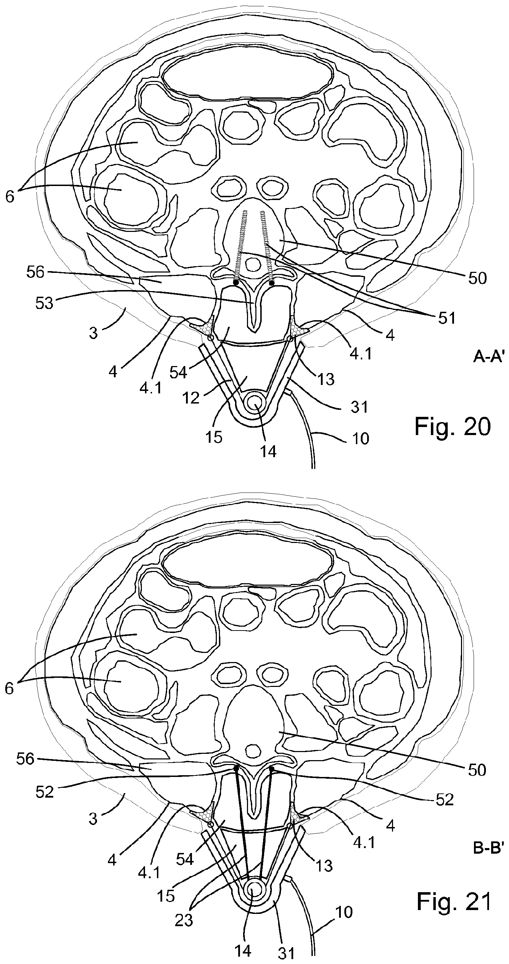

Die Darstellungen in **Fig. 20 und 21** zeigen die Bestandteile eines Kits zur Behandlung eines offenen Hautdefekts nach Art einer offenen Rückenwand. Das Kit weist neben der vorstehend beschriebenen Vorrichtung 11 zur Verminderung der Retraktion der Ränder 4.1 einer geöffneten Faszie 4 einen ersten Schwamm 54 auf, welcher derart an die Kontur des Wirbels 50 angepasst ist, dass der Dornfortsatz 53 des Wirbels 50 in eine Ausnehmung des Schwamms 54 eingreift. Ferner weist der erste Schwamm 54 Öffnungen auf, durch welche die Stützen 22, 23 der Vorrichtung 11 geführt werden können. Zudem ist ein zweiter Schwamm 15 Teil des Kits, wobei der zweite Schwamm 15 den Hautdefekt ausfüllt. Der zweite Schwamm weist vorzugsweise einen im Wesentlichen dreieckigen Querschnitt auf, so dass er den durch die Zugorgane 12 begrenzten Bereich zwischen den Rädern 4.1 der Faszie und der Aufhängung 14 ausfüllt. Der Schwamm 15 ist bevorzugt formstabil ausgebildet. Besonders bevorzugt weist der Schwamm 15 einen innenliegenden Bereich mit gegenüber seiner Oberfläche erhöhter Festigkeit oder eine innenliegende, nicht deformierbare Grundform auf. Die Zugorgane 12 und die Aufhängung 14 können insbesondere durch einen dritten Schwamm 31 abgedeckt sein. Der dritte Schwamm 31 ist bevorzugt mit den Zugorganen 12 verbindbar ausgestaltet. Der dritte Schwamm 31 kann einen Abschnitt aufweisen, dessen Form an die Außenkontur der Aufhängung 14 angepasst ist. An der außenliegenden Oberfläche des dritten Schwamms 31 kann bevorzugt ein in den Figuren nicht dargestelltes gas- und/oder flüssigkeitsdichtes Abdichtmittel 30 angeordnet werden, z. B. eine Klebefolie oder eine nicht-klebende, luftundurchlässige Folie. An den dritten Schwamm 31 kann ein Saugschlauch 10 angeschlossen werden, so dass eine vorstehend im Zusammenhang mit Fig. 2 beschriebene Unterdrucktherapie durchgeführt werden kann.

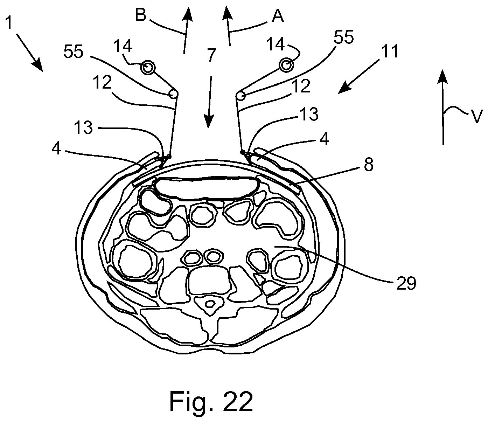

Bei den vorstehend beschriebenen Vorrichtungen 11 zur Verminderung der Retraktion der Ränder 4.1 einer Faszie 4 kann optional eine Umlenkreinrichtung 55 zur Umlenkung der Zugorgane 12 vorgesehen sein. Eine solche Umlenkeinrichtung ist exemplarisch in der **Fig. 22** gezeigt. Die Umlenkeinrichtung ist nach Art einer Stange ausgebildet, um welche das Zugorgan 12 geführt ist. Das Zugorgan 12 verläuft somit ausgehend von der Faszie 4 über die Umlenkeinrichtung 55 bis zu der Aufhängung 14.

Die vorstehend beschriebenen Kits zur Behandlung eines offenen Weichteildefekts, insbesondere einer offenen Bauchdecke 2 oder eines offenen Weichteildefekts am Rücken, mit mindestens einem Schwamm 15, 28 zum Ausfüllen eines Weichteildefekts, insbesondere einer Bauchdeckenöffnung 7 oder eines offenen Weichteildefekts am Rücken, weisen jeweils eine Vorrichtung 11 zur Verminderung der Retraktion der Ränder einer geöffneten Faszie 4 eines Patienten 1 mit einem offenen Weicheildefekt, insbesondere mit einer offenen Bauchdecke 2 oder einem offenen Weichteildefekt am Rücken, auf, über die eine Zugkraft mit einer vom Körper des Patienten weg gerichteten Kraftkomponente A₁, B₁ derart auf die Ränder der Faszie 4 aufbringbar ist, dass die Vorrichtung 11 die Ränder der Faszie 4 auf Spannung und voneinander beabstandet hält und dass weiterhin ein offener Weichteildefekt, insbesondere eine geöffnete Bauchdecke oder ein offener Weichteildefekt am Rücken, besteht.

Durch die erfindungsgemäße Vorrichtung 11 kann die Faszie 4 derart weit offen gehalten werden, dass der Körperinnenraum, insbesondere der Bauchraum 29 oder der innenliegend der Rückenwand liegende Raum, frei zugänglich ist und/oder die innenliegenden Organe 6 durch den offenen Weichteildefekt, insbesondere die Bauchdeckenöffnung 7 oder einen offenen Weichteildefekt am Rücken zumindest im Wesentlichen ungehindert austreten können. Ferner kann die Vorrichtung 11 verwendet werden, um eine Faszie 4, deren Ränder 4.1 sich bereits zurückgezogen haben, zu dehnen. Die erfindungsgemäße Vorrichtung kann verwendet werden, ohne dass eine Unterdruck-Wundtherapie durchgeführt wird.

### Bezugszeichenliste:

- 1: Patient
- 2: Bauchdecke
- 3: Haut
- 3.1: Hautrand
- 4: Faszie
- 4.1: Faszienrand
- 4.1a, 4.1b: Faszienrand
- 5: Bauchmuskel
- 6: Darm
- 7: Bauchdeckenöffnung
- 8,9: Schwamm
- 10: Saugschlauch
- 11: Vorrichtung zur Verminderung der Retraktion der Faszie
- 12: Zugorgan
- 13: Verbindungsmittel
- 14: Aufhängung
- 14.1, 14.2: Teilstücke
- 14.3: Verbindungsstelle
- 14.4: Halteelement
- 14.5: Anbindungselement
- 14.6: Distanzelement
- 15: Schwamm
- 16, 17: Befestigungseinrichtung
- 18, 19: Abstützung
- 20, 21: Halteplatte
- 22, 23: Stütze
- 22.1, 22.2: Stützenelement
- 23.1, 23.2: Stützenelement
- 24, 25: Gelenk
- 25a: Gelenkkopf
- 26: Brustbein
- 27: Vorderer Beckenring
- 28: Schwamm
- 29: Bauchraum
- 30: Abdichtmittel
- 31: Schwamm
- 32: Verbinder
- 33: Abdeckschwamm
- 34: Seitenschwamm
- 35: elastisches Element
- 36, 37: Gelenk
- 38, 39: Festellvorrichtung
- 40: Seil
- 41: Welle
- 42: Dichtung
- 43: Feststellkupplung
- 44: Betätigungselement
- 50: Wirbel
- 51: Pedikelschraube
- 52: Längsstab
- 53: Dornfortsatz
- 54: Schwamm
- 55: Umlenkeinrichtung
- 56: Rückenmuskel

- A, B: Zugkraft
- A₁, B₁, A₂, B₂: Kraftkomponente
- L: Länge
- V: ventrale Richtung
- W: laterale Richtung

## Patentansprüche

1. Vorrichtung zur Verminderung der Retraktion der Ränder (4.1) einer geöffneten Faszie (4) eines Patienten (1) mit einem offenen Weichteildefekt, insbesondere mit einer offenen Bauchdecke (2) oder einem offenen Weichteildefekt am Rücken, über die eine Zugkraft (A, B) mit einer vom Körper des Patienten (1) weg gerichteten Kraftkomponente (A₁, B₁) derart auf die Ränder (4.1) der Faszie (4) aufbringbar ist, dass die Vorrichtung (11) die Ränder (4.1) der Faszie (4) auf Spannung und voneinander beabstandet hält und dass weiterhin ein offener Weichteildefekt, insbesondere eine geöffnete Bauchdecke (2) oder ein offener Weichteildefekt am Rücken, besteht, wobei die Vorrichtung aufweist:
- zumindest ein mit der Faszie (4) verbindbares Zugorgan (12),
- eine außerhalb des Körpers des Patienten (1) dem Weichteildefekt vorgelagert anordbare Aufhängung (14), an welche das zumindest eine Zugorgan (12) anbindbar ist, und
- mehrere Stützen (22, 23), über welche die Aufhängung (14) in einer vom Körper des Patienten beabstandeten Position gehalten werden kann,
wobei die Stützen (22, 23) die Aufhängung (14) jeweils mit einer Befestigungseinrichtung (16, 17) verbinden, wobei die Befestigungseinrichtung (16, 17) entweder ein auf die Haut des Patienten (1) auflegbares Druckverteilungsmittel aufweist oder die Befestigungseinrichtung (16, 17) ein Implantat aufweist, welches mehrere Längsstangen (52) umfasst, die über Pedikelschrauben (51) mit Wirbeln des Patienten (1) verbindbar sind,
**dadurch gekennzeichnet, dass** die Vorrichtung eine Kraftmesseinrichtung zur Messung der durch das zumindest eine Zugorgan (12) auf die Faszie aufgebrachten Zugkraft aufweist.

2. Vorrichtung nach Anspruch 1, **dadurch gekennzeichnet, dass** das Zugorgan (12) als Faden und/oder als Draht und/oder als Netz ausgebildet ist.

3. Vorrichtung nach einem der vorhergehenden Ansprüche, **dadurch gekennzeichnet, dass** das Zugorgan (12) ein Federmittel aufweist.

4. Vorrichtung nach einem der vorhergehenden Ansprüche, **dadurch gekennzeichnet, dass** das Zugorgan (12) direkt oder über ein flächig ausgebildetes Verbindungsmittel (13) mit der Faszie (4) oder dem gesamten Weichteilmantel verbindbar ist.

5. Vorrichtung nach einem der vorhergehenden Ansprüche **dadurch gekennzeichnet, dass** die Zugkraft (A, B) über eine an der Aufhängung (14) angeordnete Spannvorrichtung, die insbesondere einen Kraftverstärker aufweist, einstellbar ist.

6. Vorrichtung nach einem der vorhergehenden Ansprüche, **dadurch gekennzeichnet, dass** die Aufhängung (14) vertikal entlang des Abdomens oder vertikal entlang des Rückens verlaufend anordbar ist.

7. Vorrichtung nach einem der vorhergehenden Ansprüche, **dadurch gekennzeichnet, dass** die Aufhängung (14) eine oder mehrere Stangen aufweist.

8. Vorrichtung nach einem der vorhergehenden Ansprüche, **dadurch gekennzeichnet, dass** die Länge (L) der Aufhängung (14) in einer vertikal entlang des Abdomens verlaufenden Richtung einstellbar ist.

9. Vorrichtung nach einem der vorhergehenden Ansprüche, **dadurch gekennzeichnet, dass** die Aufhängung (14) wahlweise entweder an den Patienten oder an eine patientenunabhängige Halterung, insbesondere ein Bett oder eine Liege, anbindbar ist.

10. Vorrichtung nach einem der vorhergehenden Ansprüche, **dadurch gekennzeichnet, dass** die Vorrichtung (1) mehrere mit der Faszie verbindbare Zugorgane (12) aufweist und die Kraftmesseinrichtung derart ausgestaltet ist, dass die Summe der von den Zugorganen (12) aufgebrachten Zugkräfte messbar ist.

11. Vorrichtung nach einem der vorhergehenden Ansprüche, **dadurch gekennzeichnet, dass** die Kraftmesseinrichtung einen Federkraftmesser, einen induktiver Kraftmesser, einen kapazitiver Kraftmesser, einen optischer Kraftmesser, einen Dehnungsmessstreifen, einen magnetischen Kraftmesser, einen elektromagnetischen Kraftmesser oder einen piezoelektrischen Kraftmesser aufweist.

12. Kit zur Behandlung eines offenen Weichteildefekts, insbesondere einer geöffneten Bauchdecke (2) oder eines offenen Weichteildefekts am Rücken, mit mindestens einem Schwamm (15, 28) zum Ausfüllen eines Weichteildefekts, insbesondere einer Öffnung (7) in der Bauchdecke oder eines offenen Weichteildefekts am Rücken, **gekennzeichnet durch** eine Vorrichtung (11) zur Verminderung der Retraktion der Ränder einer geöffneten Faszie (4) eines Patienten (1) nach einem der vorhergehenden Ansprüche.

13. Kit nach Anspruch 12, **dadurch gekennzeichnet, dass** die Vorrichtung (11) eine außerhalb des Körpers des Patienten (1) anordbare Aufhängung (14) aufweist, an welcher ein mit der Faszie verbindbares Zugorgan (12) anbindbar ist, und das Kit einen Schwamm (33) aufweist, dessen Kontur an die Außenkontur der Aufhängung (14) angepasst ist.

14. Kit nach einem der Ansprüche 12 oder 13, **dadurch gekennzeichnet, dass** das Kit ein Abdichtmittel (30) zum Abdichten des offenen Weichteildefekts aufweist, welches als nicht-klebende, luftundurchlässige Folie ausgebildet ist.

15. Kit nach Anspruch 14, **dadurch gekennzeichnet, dass** das Abdichtmittel eine oder mehrere Öffnungen aufweist, durch welche ein Teil der Vorrichtung (11) zur Verminderung der Retraktion der Ränder einer geöffneten Faszie (4) eines Patienten (1) hindurch geführt werden kann.

## Claims

1. Device for reducing the retraction of the edges (4.1) of an opened fascia (4) of a patient (1) having an open soft tissue defect, in particular having an open abdominal wall (2) or an open soft tissue defect on the back, by which of which a tensile force (A, B) having a force component (A₁, B₁) directed away from the body of the patient (1) can be applied to the edges (4.1) of the fascia (4) in such a way that the device (11) holds the edges (4.1) of the fascia (4) under tension and spaced apart from one another and that an open soft tissue defect, in particular an opened abdominal wall (2) or an open soft tissue defect on the back, is still present, the device having:
- at least one tensile member (12) connectable to the fascia (4),
- a suspension (14), which can be arranged outside the body of the patient (1) in front of the soft tissue defect and to which the at least one tensile member (12) can be attached, and
- a plurality of supports (22, 23), by way of which the suspension (14) can be held in a position spaced apart from the body of the patient,
the supports (22, 23) each connecting the suspension (14) to a fastening means (16, 17), either the fastening means (16, 17) having a pressure distribution means which can be laid on the skin of the patient (1) or the fastening means (16, 17) having an implant which comprises a plurality of longitudinal bars (52) which can be connected to vertebrae of the patient (1) by way of pedicle screws (51), **characterised in that** the device has a force measurement device for measuring the tensile force applied to the fascia by the at least one tensile member (12).

2. Device according to claim 1, **characterised in that** the tensile member (12) is formed as a thread and/or as a wire and/or as a net.

3. Device according to any of the preceding claims, **characterised in that** the tensile member (12) has a spring means.

4. Device according to any of the preceding claims, **characterised in that** the tensile member (12) can be connected to the fascia (4) or to the entire soft tissue mantle, directly or via a connection means (13) that is formed planar.

5. Device according to any of the preceding claims, **characterised in that** the tensile force (A, B) is adjustable by way of a tensioning device, which is arranged on the suspension (14) and which in particular has a force amplifier.

6. Device according to any of the preceding claims, **characterised in that** the suspension (14) can be arranged extending vertically along the abdomen or vertically along the back.

7. Device according to any of the preceding claims, **characterised in that** the suspension (14) has one or more bars.

8. Device according to any of the preceding claims, **characterised in that** the length (L) of the suspension (14) can be adjusted in a direction extending vertically along the abdomen.

9. Device according to any of the preceding claims, **characterised in that** the suspension (14) can be attached selectively either to the patient or to a patient-independent mounting, in particular a bed or a stretcher.

10. Device according to any of the preceding claims, **characterised in that** the device (1) has a plurality of tensile members (12) connectable to the fascia, and the force measurement device is configured in such a way that the sum of the tensile forces applied by the tensile members (12) can be measured.

11. Device according to any of the preceding claims, **characterised in that** the force measurement device has a spring force gauge, an inductive force gauge, a capacitive force gauge, an optical force gauge, a strain gauge, a magnetic force gauge, an electromagnetic force gauge or a piezoelectric force gauge.

12. Kit for treating an open soft tissue defect, in particular an opened abdominal wall (2) or an open soft tissue defect on the back, comprising at least one sponge (15, 28) for filling out a soft tissue defect, in particular an opening (7) in the abdominal wall or an open soft tissue defect on the back, **characterised by** a device (11) for reducing the retraction of the edges of an opened fascia (4) of a patient (1) according to any of the preceding claims.

13. Kit according to claim 12, **characterised in that** the device (11) has a suspension (14), which can be arranged outside the body of the patient (1) and to which a tensile member (12) connectable to the fascia can be attached, and the kit has a sponge (33), the contour of which is adapted to the outer contour of the suspension (14).

14. Kit according to either claim 12 or claim 13, **characterised in that** the kit has a sealing means (30) for sealing the open soft tissue defect, which is formed as a non-adhesive, air-impermeable film.

15. Kit according to claim 14, **characterised in that** the sealing means has one or more openings through which part of the device (11) for reducing the retraction of the edges of an opened fascia (4) of a patient (1) can be passed.

## Revendications

1. Dispositif de réduction de la rétraction des bords (4.1) d'un fascia ouvert (4) d'un patient (1) présentant un défaut d'ouverture de tissu mou, en particulier une paroi abdominale (2) ouverte ou un défaut d'ouverture de tissu mou dans le dos, par le biais duquel une force de traction (A, B) ayant une composante de force (A1, B1) orientée dans la direction opposée au corps du patient (1) peut être appliquée sur les bords (4.1) du fascia (4) de telle manière que le dispositif (11) maintient les bords (4.1) du fascia (4) tendus et espacés les uns des autres et qu'un défaut d'ouverture de tissu mou, en particulier une paroi abdominale (2) ouverte ou un défaut d'ouverture de tissu mou dans le dos, subsiste, le dispositif comportant :
- au moins un organe de traction (12) pouvant être relié au fascia (4),
- un système de suspension (14) pouvant être disposé à l'extérieur du corps du patient (1) devant le défaut de tissu mou, auquel l'au moins un organe de traction (12) peut être relié, et
- plusieurs appuis (22, 23), par le biais desquels le système de suspension (14) peut être maintenu dans une position espacée du corps du patient,
les appuis (22, 23) reliant le système de suspension (14) respectivement à un moyen de fixation (16, 17), le moyen de fixation (16, 17) comportant un moyen de distribution de la pression pouvant être posé sur la peau du patient (1) ou le moyen de fixation (16, 17) comportant un implant, qui comprend plusieurs tiges longitudinales (52) qui peuvent être reliées à des vertèbres du patient (1) par le biais de vis pédiculaires (51),
**caractérisé en ce que** le dispositif comporte un moyen de mesure de la force pour mesurer la force de traction appliquée sur le fascia par l'au moins un organe de traction (12) .

2. Dispositif selon la revendication 1, **caractérisé en ce que** l'organe de traction (12) se présente sous la forme d'un fil et/ou d'un fil métallique et/ou d'un filet.

3. Dispositif selon l'une des revendications précédentes, **caractérisé en ce que** l'organe de traction (12) comporte un moyen formant ressort.

4. Dispositif selon l'une des revendications précédentes, **caractérisé en ce que** l'organe de traction (12) peut être relié au fascia (4) ou à l'ensemble de l'enveloppe de tissu mou directement ou par l'intermédiaire d'un moyen de liaison (13) réalisé plan.

5. Dispositif selon l'une des revendications précédentes, **caractérisé en ce que** la force de traction (A, B) peut être réglée par le biais d'un dispositif tendeur disposé sur le système de suspension (14), lequel dispositif tendeur comporte en particulier un amplificateur de force.

6. Dispositif selon l'une des revendications précédentes, **caractérisé en ce que** le système de suspension (14) peut être disposé s'étendant verticalement le long de l'abdomen ou verticalement le long du dos.

7. Dispositif selon l'une des revendications précédentes, **caractérisé en ce que** le système de suspension (14) comporte une ou plusieurs tiges.

8. Dispositif selon l'une des revendications précédentes, **caractérisé en ce que** la longueur (L) du système de suspension (14) peut être réglée dans une direction s'étendant verticalement le long de l'abdomen.

9. Dispositif selon l'une des revendications précédentes, **caractérisé en ce que** le système de suspension (14) peut être relié au choix à un patient ou à un support indépendant du patient, en particulier un lit ou un couchage.

10. Dispositif selon l'une des revendications précédentes, **caractérisé en ce que** le dispositif (1) comporte plusieurs organes de traction (12) pouvant être reliés au fascia et le moyen de mesure de la force est conçu de telle manière que la somme des forces de traction appliquées par les organes de traction (12) peut être mesurée.

11. Dispositif selon l'une des revendications précédentes, **caractérisé en ce que** le moyen de mesure de la force comporte un dynamomètre à ressort, un dynamomètre inductif, un dynamomètre capacitif, un dynamomètre optique, une jauge extensométrique, un dynamomètre magnétique, un dynamomètre électromagnétique ou un dynamomètre piézoélectrique.

12. Kit de traitement d'un défaut d'ouverture de tissu mou, en particulier d'une paroi abdominale ouverte (2) ou d'un défaut d'ouverture de tissu mou dans le dos, comprenant au moins une éponge (15, 28) destinée à remplir un défaut de tissu mou, en particulier une ouverture (7) dans la paroi abdominale ou un défaut d'ouverture de tissu mou dans le dos, **caractérisé par** un dispositif (11) de réduction de la rétraction des bords d'un fascia (4) ouvert d'un patient (1) selon l'une des revendications précédentes.

13. Kit selon la revendication 12, **caractérisé en ce que** le dispositif (11) comporte un système de suspension (14) pouvant être disposé à l'extérieur du corps du patient (1), auquel peut être relié un organe de traction (12) pouvant être relié au fascia, et le kit comporte une éponge (33), dont le contour est adapté au contour extérieur du système de suspension (14).

14. Kit selon l'une des revendications 12 et 13, **caractérisé en ce que** le kit comporte un moyen d'obturation (30) pour obturer le défaut d'ouverture de tissu mou, lequel moyen d'obturation se présente sous la forme d'un film non collant imperméable à l'air.

15. Kit selon la revendication 14, **caractérisé en ce que** le moyen d'obturation comporte une ou plusieurs ouvertures, par lesquelles une partie du dispositif (11) de réduction de la rétraction des bords d'un fascia (4) ouvert d'un patient (1) peut être passée.
